# EUROPEAN PATENT APPLICATION

(11) **EP 4 803 067 A1**
(43) Date of publication of application: **09.09.2026**
(21) Application number: 25161670.2
(22) Date of filing: 04.03.2025
(51) Int. Cl.: A61K 6/887, A61K 6/30, A61K 6/62, A61K 6/893

(54) **3D PRINTABLE MATERIALS WITH HIGH FRACTURE WORK AND FRACTURE TOUGHNESS**

(71) Applicant: Ivoclar Vivadent AG, 9494 Schaan (LI)
(72) Inventor: Rist, Kai, 6800 Feldkirch (AT); Catel, Yohann, 9475 Sevelen (CH); Lamparth, Iris, 9472 Grabs (CH); Grob, Benjamin, 9470 Buchs (CH)
(74) Representative: Uexküll & Stolberg

(57) **Abstract**

Radically polymerizable urethane di(meth)acrylate macromonomer according to Formula I and radically polymerizable dental material, which comprises at least one radically polymerizable urethane di(meth)acrylate macromonomer according to Formula I, at least one ABA block copolymer, at least one monofunctional, radically polymerizable monomer and an initiator for the radical polymerization:

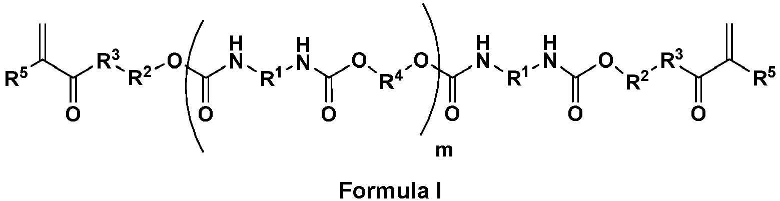

## Description

The present invention relates to radically polymerizable compositions which can be processed by additive processes such as 3D printing and which have high fracture work and fracture toughness in combination with high flexural strength and modulus. The materials are particularly suitable as dental material for the production of dental moldings, such as artificial teeth, dental prostheses, inlays, onlays, splints (bite splints), crowns, bridges, veneering materials, aligners and orthodontic appliances.

Radically polymerizable dental materials typically comprise a mixture of monomers, initiator components, stabilizers, fillers and pigments. The monomers usually comprise high-viscosity dimethacrylates such as 2,2-bis[4-(2-hydroxy-3-methacryloyloxy-propyl)phenyl]propane (bis-GMA) and 1,6-bis-[2-methacryloyloxyethoxycarbonyl-amino]-2,4,4-trimethylhexane (UDMA) and low-viscosity dimethacrylates such as bis-methacryloyloxymethyltricyclo[5.2.1.]decane (TCDMA), decanediol-1,10-dimethacrylate (D₃MA) and triethylene glycol dimethacrylate (TEGDMA). For some applications, monofunctional monomer such as methyl methacrylate (MMA) can be added.

In additive manufacturing processes, which are also referred to as generative manufacturing processes, 3D shaped bodies are generated in layers from polymerizable materials starting from a CAD dataset, wherein single lines or dots of material are deposited and cured or whole layers are cured by controlled exposure to light.

Materials for the additive production of dental shaped bodies should have a low viscosity, high transparency and good mechanical properties after curing.

US 10,562,995 B2 discloses polymerization resins for the stereolithographic production of dental prostheses which are based on mixtures of aromatic di(meth)acrylates, which have no OH or COOH groups, with (meth)acrylic monomers, which contain at least one OH or COOH group.

US 10,568,814 B2 discloses photopolymerizable compositions for the production of artificial teeth and denture bases by 3D printing, which are said to have a high flexural strength and a high modulus of elasticity after curing. The materials are based on a mixture of ethoxylated bisphenol A dimethacrylate, monofunctional methacrylates and urethane dimethacrylates.

EP 3 020 361 A1 relates to curable compositions for additive manufacturing processes, which contain radically polymerizable polysiloxanes and disiloxanes. The materials are said to be characterized by a high dimensional stability and improved biocompatibility.

EP 3 494 954 A1 discloses photopolymerizable compositions for the production of dental prostheses which contain a mixture of aromatic acrylates with a molar mass of from 200 to 800 g/mol and at least one further (meth)acrylate, which can contain aromatic and non-aromatic rings. The materials are said to be characterized by a good Charpy fracture toughness.

EP 3 564 206 A1 discloses (meth)acryloxy-substituted benzoic acid esters, which are said to be suitable as reactive diluents for additive manufacturing processes.

WO 2014/078537 A1 discloses resin mixtures for the production of dental shaped bodies by 3D printing processes based on mono- and multifunctional methacrylates, which contain silicone acrylate-based impact modifiers with a core-shell structure for improving the impact resistance and fracture toughness.

US 2018/0000570 A1 relates to building materials based on mono- and multifunctional (meth)acrylates for the additive production of dental components. The building materials contain rubber particles based on silicone acrylic with a core-shell structure (product S2006 from Mitsubishi Rayon Co.) as impact modifiers and oligomers, which are prepared by reacting trimethyl 1,6-diisocyanate, bisphenol A propoxylate and 2-hydroxyethyl methacrylate (HEMA). The cured components are said to have good mechanical and physical properties as well as a good biocompatibility.

US 10,299,896 B2 and US 2019/0053883 A1 disclose dental components produced by additive processes which have at least two layers of building materials with different compositions. One layer is formed by a material which contains oligomers, which are obtained by reacting intermediate products having terminal isocyanate groups with hydroxyl-based methacrylates, a polymerizable acrylic compound and an impact modifier. At least one further layer is formed by a material which contains a urethane monomer, a glycol dimethacrylate and filler. The combination of materials with different mechanical and physical properties is said to be advantageous for adapting the components to different requirements. Commercially available polymers with a core-shell structure, such as e.g. the product M570 from Kaneka, are used as impact modifiers.

EP 3 564 282 A1 discloses curable compositions for high-temperature photopolymerization processes which contain an oligomeric urethane dimethacrylate as glass transition temperature modifier, a (poly)carbonate-(poly)urethane dimethacrylate as toughness modifier and optionally core-shell particles. They are said to have good thermomechanical properties and good biocompatibility and to be suitable for the production of orthodontic appliances.

A major disadvantage of impact modifiers with a core-shell structure is that core-shell polymers (CSP) significantly reduce the transparency of the materials, which has a detrimental effect on the stereolithographic building process and, in the case of dental materials, it is also undesirable for aesthetic reasons.

WO 2016/071811 A1 discloses dental materials containing a polyrotaxane compound comprising a polymer chain onto which one or more cyclodextrin rings are slipped. The polymer chain is selected from polyethylene glycol (PEG), polypropylene glycol (PPG), a PEG-PPG block copolymer or a polydimethylsiloxane. The materials are said to be suitable for producing three-dimensional dental objects by stereolithography.

EP 2 492 289 A1 discloses dental materials comprising an acrylic block copolymer with at least one hard segment A and at least one soft segment B, a polymerizable monomer (b), such as urethane di(meth)acrylate, and an initiator (c). The materials are said to be suitable as temporary cement for implant use and as mobile tooth-fixing material.

EP 4 085 893 A1 discloses a radically polymerizable dental material, which contains at least one ABA or AB block copolymer, preferably at least one monofunctional, radically polymerizable monomer (a) and preferably at least one radically polymerizable urethane di(meth)acrylate telechel (b). The material can be processed by additive processes, has high transparency in combination with a good fracture toughness and high fracture work.

The object of the invention is to provide materials that can be processed by additive processes and in particular by 3D printing with improved fracture work and fracture toughness in combination with high flexural strength and modulus. The materials are to be particularly suitable for the production of dental moldings such as artificial teeth, dental prostheses, inlays, onlays, splints (bite splints), crowns, bridges, veneering materials, aligners and orthodontic appliances. In addition, they should exhibit a low viscosity and good mechanical properties after storage in water as well as a good biocompatibility.

According to the invention, this object is achieved by radically polymerizable dental materials, which contain at least one radically polymerizable urethane di(meth)acrylate macromonomer according to Formula I and in particular by dental materials which additionally comprise at least one aromatic, bicyclic or tricyclic mono(meth)acrylate and an ABA block copolymer.

The **urethane di(meth)acrylate macromonomers** of Formula I have the following structure: 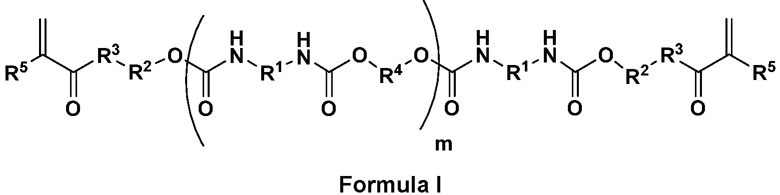 wherein
- R¹ is: 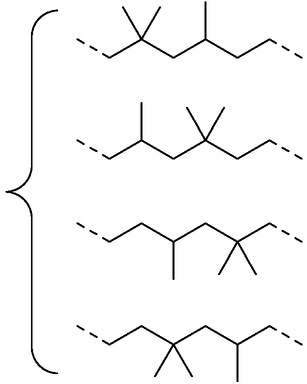
- R²: is a C₂-C₁₁, preferably a C₂-C₈ and most preferably a C₂-C₆ alkylidene group, that can be branched or linear and that can be interrupted by one or more, preferably a single, O atoms,
- R³: O, NH or NR⁶, in which R⁶ is a linear and branched C₁-C₆ alkyl group,
- R⁴: is one of the following groups: 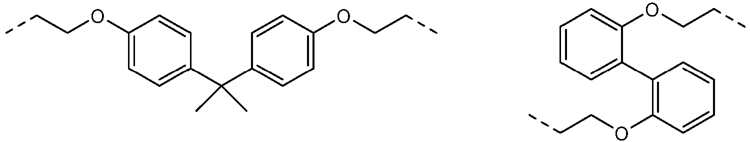 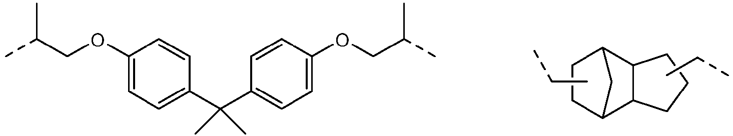 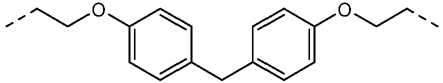
- R⁵: is H or preferably CH₃,
- m: is a value from 0.5 to 3.

The residue R¹ can have any of the above meanings and the macromonomers of Formula I are usually a mixture of isomers in which R¹ has different meanings.

For the synthesis of the macromonomers of Formula **I,** the reagents are preferably used in molar ratios, which would ideally yield a single macromonomer with a single, defined number of repeating units. However, it is difficult to control the reaction in that way, that only such a single macromonomer is formed. Instead, a mixture of i macromonomers with various *mᵢ* values is formed. This reaction mixture is preferably used without separation of the individual homologs, and m is the number average of all *mᵢ* of the mixture. This number average is calculated according to the formula $m = \frac{{\sum }_{i = 0} {N}_{i} {m}_{i}}{{\sum }_{i = 0} {N}_{i}}$ where *mᵢ* is the number of repeating units for a specific macromonomer *i* and *Nᵢ* is the number of the macromonomer molecules with *mᵢ* repeating units according to Formula I.

In that way, *m* can be used to characterize such macromonomers and to calculate the equivalents of reagents for synthesis. *m* is a value of from 0.5 to 3.0, preferably 0.6 to 2.0, more preferably 0.7 to 1.6, and most preferably 0.9 to 1.6. In a particularly preferred embodiment *m* is 0.7, in a more preferred embodiment *m is* 1.5, and in a most preferred embodiment *m is* 1.0.

During the synthesis of the macromonomers of Formula I, a mixture of compounds is generally formed in which n has different values. The reagents used for the synthesis of the macromonomers are preferably used in a molar ratio which corresponds to n = 1or 2, preferably 1. However, it is difficult to control the reaction so that only compounds are formed in which n has a particular value. Usually, compounds wherein n has other meanings are formed as by-products. This reaction mixture is preferably used without separation of the individual homologs, and n is the average value of the mixture.

The urethane di(meth)acrylate macromonomers of Formula I preferably have a number-average molar mass of from 800 to 2000 g/mol, more preferably 800 to 1800 g/mol and most preferably 800 to 1600 g/mol.

Unless otherwise stated, the molar mass of macromonomers, oligomers and polymers herein is the number-average molar mass, the absolute values of which can be determined using the known methods of freezing point depression (cryoscopy), boiling point elevation (ebullioscopy) or from the decrease in the vapor pressure (vapor pressure osmometry). The number-average molar mass of macromonomers, oligomers and polymers is preferably determined by means of gel permeation chromatography (GPC). This is a relative method in which the molecules are separated on the basis of their size, more specifically on the basis of their hydrodynamic volume. The absolute molar mass is determined through calibration with known standards.

According to a particularly preferred embodiment of the present invention, the variables of Formula I have the following meanings:
- R¹ is: 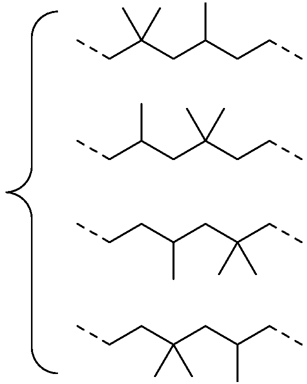
- R²: is a linear C₂-C₆, preferably a linear C₂-C₄ alkylidene group,
- R³: is O,
- R⁴: is one of the following groups: 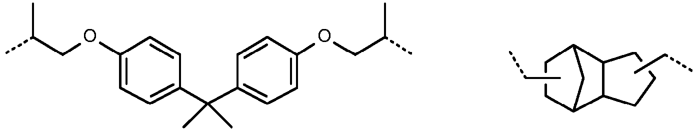
- R⁵: is CH₃,
- m: is a value from 0.5 to 3.0, preferably 0.6 to 2.0, more preferably 0.7 to 1.6 and most preferably 0.9 to 1.6.

All formulae shown herein extend only to those compounds which are compatible with the theory of chemical valence. The indication that a radical is interrupted e.g. by one or more oxygen atoms is to be understood to mean that these atoms are inserted in each case into the carbon chain of the radical. These atoms are thus bordered on both sides by C atoms and cannot be terminal. C₁ radicals cannot be branched or interrupted. Corresponding to the usual nomenclature, by aromatic hydrocarbon radicals is also meant those radicals which contain aromatic and non-aromatic groups.

In all cases described herein, the preferred, more preferred and most preferred definitions given for the individual variables can each be selected independently of one other. Compounds in which all the variables have the preferred, particularly preferred and quite particularly preferred definitions are naturally particularly suitable according to the invention.

The urethane di(meth)acrylate macromonomers of Formula I are preferably obtained by reacting a diisocyanate with a diol (HO-R⁴-OH) and then reacting the α,ω-isocyanate-functionalized urethane macromonomers with a compound bearing one hydroxy group and one radically polymerizable group, preferably a (meth)acrylate, (meth)acrylamide or (N-alkyl)(meth)acrylamide group, and preferably with HEMA or HPMA. According to a particularly preferred embodiment of the present invention, (*m*+1) equivalents (eq.) of trimethylhexamethylene-1,6-diisocyanate are reacted with *m* moles of a diol (HO-R⁴-OH). The thereby formed α,ω-isocyanate-functionalized urethane oligomers are further reacted with 1 eq. of a hydroxyalkyl methacrylate ("end-capping"). R⁴ is as defined above. Preferred diols of the formula HO-R⁴-OH are tricyclo[5.2.1.0^{2,6}]decanedimethanol, 1,1'-isopropylidenebis(p-phenyleneoxy)di-propan-2-ol, 4,4'-Isopropylidenebis(2-phenoxyethanol), 2,2'-[methylenebis(4,1-phenyleneoxy)]bis[ethanol] and 2-[2'-(2-hydroxyethoxy)biphenyl-2-yloxy]ethanol. The commercially available trimethylhexamethylene-1,6-diisocyanate (TDMI) is a mixture of 2,2,4-trimethylhexamethylene-1,6-diisocyanate and 2,4,4-trimethylhexamethylene-1,6-diisocyanate, which is preferably used without separation.

To calculate *m* of a macromonomer resulting from synthesis according to the aforementioned method using a specific mixture of reagents, the following formula can be used: $m = \frac{{mol}_{diol}}{{mol}_{TMDI} - {mol}_{diol}}$ wherein *mol_{diol}* is the molar amount of the used diol and *mol_{TMDI}* is the molar amount of the used diisocyanate.

As an example, the particularly preferred macromonomer DMA1 can be synthesized by reacting the diol tricyclo[5.2.1.0^{2,6}]decanedimethanol with TDMI and end-capping the intermediate with 2-hydroxyethyl methacrylate according to the following pathway:

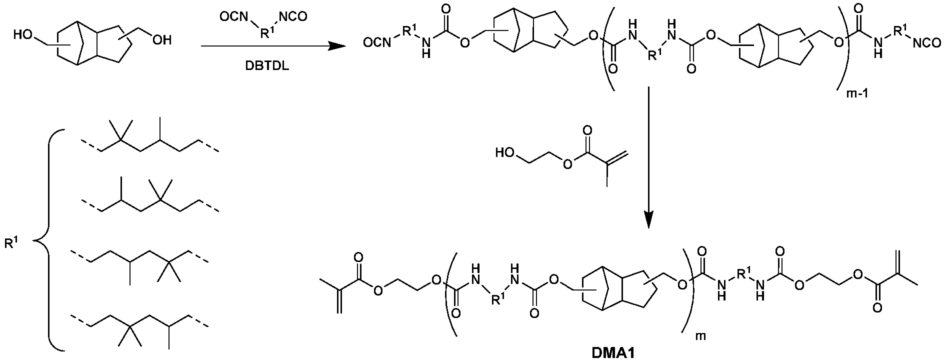

In this example, the reaction between alcohol and isocyanate is catalyzed with dibutyltin dilaurate (DBTDL).

Since all isocyanate groups, those of TMDI and those of the already formed isocyanate-diol-adducts, have a similar reactivity, the synthesis yields a mixture of macromonomers with different molecular weights characterized by a number average of m. This macromonomer mixture is preferably used without further purification or separation.

The invention also relates to radically polymerizable dental materials, which comprise at least one radically polymerizable urethane di(meth)acrylate macromonomer according to Formula I, at least one (PCL)_{q}-b-(PDMS)ᵣ-b-(PCL)_{q} block copolymer, at least one monofunctional, radically polymerizable monomer and an initiator for the radical polymerization:
The dental materials according to the invention preferably have the following composition:
(a) 15 to 75 wt. %, preferably 20 to 70 wt.% and more preferably 25 to 65 wt.% of at least one aromatic, bicyclic or tricyclic **mono(meth)acrylate,**
(b) 15 to 75 wt. %, preferably 20 to 70 wt.% and more preferably 25 to 65 wt.% of at least one **urethane di(meth)acrylate macromonomer** according to Formula I,
(c) 0 to 30 wt.%, preferably 0 to 20 wt.% and more preferably 0 to 10 wt.% of one or more **di(meth)acrylate monomers,**
(d) 0.1 to 10 wt.%, especially 2 to 10 wt.%, preferably 3 to 9 wt.% and more preferably 4 to 8 wt.% of at least one (PCL)_{q}-b-(PDMS)ᵣ-b-(PCL)_{q} **block copolymer** having a number-average molar mass of 2000 to 10000 g/mol, wherein (PCL)_{q} represents polycaprolactone, which is made up of q caprolactone monomer units, and (PDMS)ᵣ represents poly(dimethylsiloxane), which is made up of r dimethylsiloxane monomer units and the letter b stands for *block,* and
(e) 0.1 to 3.0 wt.%, preferably 0.3 to 2.5 wt.% and more preferably 0.5 to 2.0 wt.% of at least one **initiator** for the radical polymerization.

Unless otherwise stated, all percentages by weight herein relate to the total mass of the material.

Herein, (meth)acrylate represents acrylate, methacrylate or a mixture thereof.

Preferred dental materials are those in which the preferred, more preferred and most preferred components defined herein are used for each component. The preferred components can be selected independently of each other, whereby dental materials in which all components are preferred, particularly preferred and most preferred are naturally particularly suitable according to the invention.

The dental materials according to the invention preferably contain as **component (a)** at least one aromatic, bicyclic or tricyclic mono(meth)acrylate, preferably monoacrylate, of Formula II 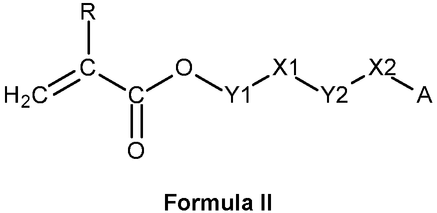 in which the variables have the following meanings:
- A: is an aromatic group with 6 to 15 carbon atoms or a bicyclic or tricyclic aliphatic group with 7 to 10 carbon atoms, wherein A can be unsubstituted or substituted by one or more C₁-C₅ alkyl groups, C₁-C₅ alkoxy groups and/or chlorine atoms;
- R: methyl or preferably H;
- X¹, X²: independently of each other are in each case absent or an ether, ester or urethane group, wherein X¹ is absent if Y¹ is absent and wherein X² is absent if Y² is absent;
- Y¹, Y²: independently of each other are in each case absent or a branched or preferably linear aliphatic hydrocarbon radical with 1 to 10 carbon atoms, which can be interrupted by 1 to 3 oxygen atoms.

Preferred aromatic groups A are benzene, biphenyl and 2,2-diphenylpropane:

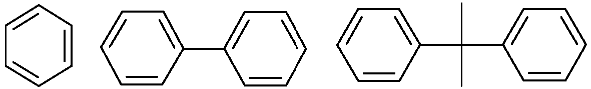

Preferred bicyclic aliphatic groups A are bicyclo[4.4.0]decane, bicyclo[4.3.0]nonane, bicyclo[2.2.2]octane and bicyclo[2.2.1]heptane:

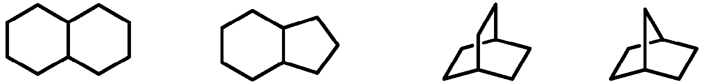

A preferred tricyclic aliphatic group A is tricyclo[5.2.1.0^{2,6}]decane:

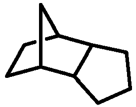

Preferred aromatic mono(meth)acrylates (a) are 2-phenoxyethyl (meth)acrylate, 2-(o-biphenyloxy)ethyl (meth)acrylate, 2-hydroxy-3-phenoxypropyl (meth)acrylate, 2-[(benzyloxycarbonyl)-amino]-ethyl (meth)acrylate, 2-[(benzylcarbamoyl)-oxy]-ethyl (meth)acrylate, 1-phenoxypropan-2-yl (meth)acrylate and 2-(p-cumylphenoxy)-ethyl (meth)acrylate. Particularly suitable aromatic mono(meth)acrylates are 2-phenoxyethyl (meth)acrylate, 2-(o-biphenyloxy)ethyl (meth)acrylate, 2-hydroxy-3-phenoxypropyl (meth)acrylate, 2-[(benzyloxycarbonyl)amino]-ethyl (meth)acrylate, 1-phenoxy-propan-2-yl (meth)acrylate, 2-(benzyloxy)ethyl (meth)acrylate, 3-phenoxybenzyl (meth)acrylate, phenoxypropyl (meth)acrylate, 2-benzyloxyethyl (meth)acrylate, 2-benzoyloxyethyl (meth)acrylate, 2-(meth)acryloyloxybenzoic acid methyl ester, 2-phenylethyl (meth)acrylate and/or 2-(p-cumylphenoxy)ethyl (meth)acrylate.

Preferred bi- or tricyclic mono(meth)acrylates (a) are octahydro-4,7-methano-1H-indenyl)methyl (meth)acrylate, octahydro-4,7-methano-1H-indenyl) (meth)acrylate, isobornyl (meth)acrylate or a mixture thereof. Acrylates are preferred over methacrylates.

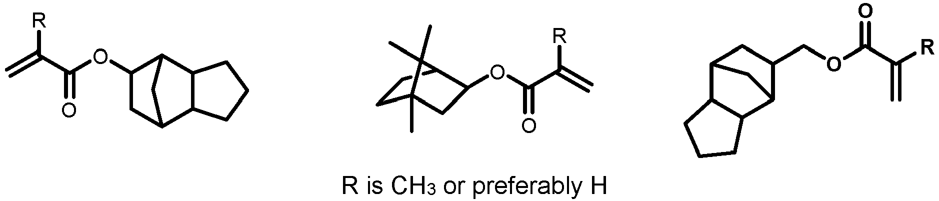

The most preferred acrylate monomers (a) are (octahydro-4,7-methano-1H-indenyl)methyl acrylate (OMIMA), isobornyl acrylate (IBOA) or a mixture thereof.

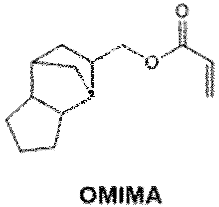

The aromatic, bicyclic or tricyclic mono(meth)acrylates of Formula II used according to the invention are characterized by a good radical polymerizability. In addition, the polymers of these mono(meth)acrylates have a comparatively low polymerization shrinkage and good mechanical properties. Because of their relatively high molar mass (150 to 350 g/mol) and their relatively non-polar structure, the mono(meth)acrylates of Formula II also have a low volatility and a comparatively low viscosity.

The dental materials according to the invention contain as **component (b)** at least one urethane di(meth)acrylate macromonomer according to Formula I. Component (b) contains two radically polymerizable groups and thus acts as crosslinker during the polymerization of the materials according to the invention, that is to say it leads to the formation of polymer networks. Because of the relatively high molar mass of component (b), polymers with a low network density and low polymerization shrinkage are obtained.

The urethane di(meth)acrylate macromonomers according to the invention are characterized by a good radical polymerizability. It was surprisingly found that the urethane di(meth)acrylate macromonomers of Formula I significantly improve the fracture toughness of radically polymerizable materials, in particular if combined with a (PCL)_{q}-b-(PDMS)ᵣ-b-(PCL)_{q} block copolymer and preferably also a mono(meth)-acrylate of Formula II. Materials comprising a macromonomer of Formula I, a (PCL)_{q}-b-(PDMS)ᵣ-b-(PCL)_{q} block copolymer and preferably also a mono(meth)acrylate of Formula II exhibit a significantly improved fracture toughness (significantly higher maximum stress intensity factor (Kmax)) and total fracture work (Wf), combined with a high flexural strength and modulus.

To further adjust the crosslinking density and to influence the mechanical properties of the polymers, the dental materials according to the invention can additionally contain further **di(meth)acrylate monomers (c)** in addition to components (a) and (b). Dimethacrylate monomers are preferred as component (c).

Preferred di(meth)acrylates (c) are bisphenol A dimethacrylate (bis-GMA, an addition product of methacrylic acid and bisphenol A diglycidyl ether), ethoxylated or propoxylated bisphenol A dimethacrylate, such as e.g. the bisphenol A dimethacrylate SR-348C (Sartomer) with 3 ethoxy groups, 2,2-bis[4-(2-methacryloxypropoxy)phenyl]-propane (UDMA, an addition product of HEMA and TMDI), V380 (an addition product of a mixture of 0.7 eq. 2-hydroxyethyl methacrylate and 0.3 eq. 2-hydroxypropyl methacrylate with 1 eq. α,α,α',α'-tetramethyl-m-xylylene diisocyanate), bis(meth-acryloyloxymethyl)tricyclo-[5.2.1.0^{2,6}]decane (DCP), di-, tri- or tetraethylene glycol dimethacrylate, as well as glycerol dimethacrylate, 1,4-butanediol dimethacrylate, 1,10-decanediol dimethacrylate (D₃MA) and 1,12-dodecanediol dimethacrylate.

The di(meth)acrylate monomers (c) are characterized by a relatively low molar weight. Di(meth)acrylates (c) with a molar weight in the range of from 200 to 800 g/mol, preferably 220 to 650 g/mol, are preferred according to the invention. Due to the low molar weight in comparison with the urethane di(meth)acrylate macromonomers (b), the di(meth)acrylate monomers (c) bring about a relatively strong crosslinking of the polymers and thus result in a high network density, which can have a disadvantageous effect on the fracture toughness. The proportion of further di(meth)acrylates is therefore limited to a maximum of 30 wt.%, preferably a maximum of 10 wt.%. According to a particularly preferred embodiment, the dental materials according to the invention contain exclusively the urethane di(meth)acrylate macromonomers (b) as crosslinker.

Moreover, the dental materials according to the invention can contain further mono(meth)acrylates in addition to component (a). The proportion of further mono(meth)acrylates preferably lies below 10 wt.%, wherein materials which contain no further mono(meth)acrylates are particularly preferred.

The dental materials according to the invention contain as **component (d)** at least one ABA block copolymer according to the formula (PCL)_{q}-b-(PDMS)ᵣ-b-(PCL)_{q} having a number-average molar mass of from 2000 to 10000 g/mol, preferably 3000 to 9000 g/mol and more preferably 3500 to 7000 g/mol. The variables q and r preferably have the following meanings:
- q: in each case is a number from 4 to 30, preferably 7 to 20, and
- r: is a number from 15 to 60, preferably 20 to 45 and
- r/q: is within a range of 1.75 to 4.
(PCL)_{q} represents polycaprolactone, which is made up of q caprolactone monomers, and (PDMS)ᵣ represents poly(dimethylsiloxane), which is made up of r dimethylsiloxane monomers. The letter b stands for *block.* Both q variables are preferably identical.

The block copolymers can be prepared, starting from a bisamino or bishydroxy terminated polydimethylsiloxane block, by ring-opening polymerization of ε-caprolactone (CL) in the presence of a catalyst. As an example, a bisaminopropyl terminated polydimethylsiloxane block can be used and bis(2-ethylhexanoate)tin, Sn(EH)₂, can be selected as catalyst:

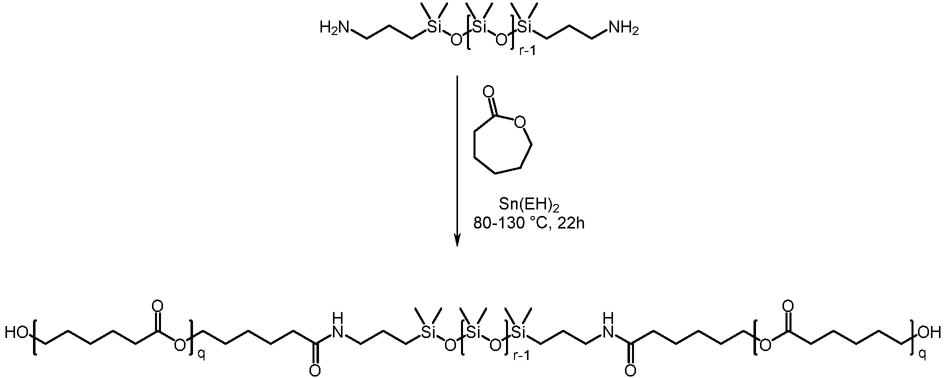

The block copolymers according to the invention are ABA triblock copolymers wherein the A block is a polymer of caprolactone and the B block is a polymer of dimethylchlorosilane. The A blocks are miscible with the resin matrix, i.e. the mixture of constituents (a) to (c), and the B block is not miscible with the resin matrix. Here, the miscibility is meant in the sense of thermodynamics in relation to the single-phase state. According to this, by a miscible polymer block is meant a polymer block consisting of a monomer, the homopolymer of which is soluble in the resin matrix, with the result that the mixture has a transparency of at least 95%. In contrast, if the mixture is cloudy or opaque, i.e. the transparency is lower than 95%, then the homopolymer, and thus the corresponding polymer block, is not miscible with the resin matrix. The transparency is measured in transmission (D65) in accordance with the ISO 10526:1999 standard on 1 mm-thick test pieces polished to high gloss using a spectrophotometer, e.g. using a Konika-Minolta CM-5-type spectrophotometer.

The block copolymers bring about a significant improvement in the fracture toughness of the materials according to the invention after curing. It is assumed that the immiscibility of the B block of the block copolymers with the remaining constituents of the compositions according to the invention brings about a microphase separation and thus the formation of morphologies at the nanoscale level. Here, the macromolecules of the ABA block copolymers form spherical or worm-like phases in the monomer resin or, during the curing, by self-assembly, which phases can interact with crack tips, that is to say crack tips meet the phases and the fracture energy is distributed into the phases such that the cracks do not migrate further through the material and do not increase in size. The growth of a crack can be observed under an electron microscope in transparent materials. In fracture mechanics, the frontmost part of the crack is called the crack tip.

The block copolymer or block copolymers are preferably used in an amount of from 0.1 to 10 wt.%, especially 2 to 10 wt.%, more preferably in an amount of from 3 to 9 wt.% and most preferably in an amount of from 4 to 8 wt.%, based on the total weight of the dental material.

It was known that block copolymers of the formula (PCL)_{q}-b-(PDMS)ᵣ-b-(PCL)_{q} improve the fracture toughness of the polymer networks, without impairing the transparency. It was now surprisingly found that the stress intensity factor Kₘₐₓ and the total fracture work Wf can be further substantially improved by combining these block copolymers with specific urethane di(meth)acrylate macromonomers, that is the urethane di(meth)acrylate macromonomers of Formula I. Best results are achieved if the block copolymers (d) and the urethane di(meth)acrylate macromonomers of Formula I are combined with the monomers (octahydro-4,7-methano-1H-indenyl)methyl acrylate (OMIMA), isobornyl acrylate (IBOA) or a mixture thereof.

The block copolymers used according to the invention can easily be homogeneously mixed with the remaining components of the materials. They can be incorporated well into resin mixtures, with the result that it is possible to match the materials to the planned application and to set the desired fracture toughness and fracture work without problems. Moreover, they bring about only a relatively small increase in viscosity.

The dental materials according to the invention contain as **component (e)** at least one initiator for the radical polymerization, preferably a photoinitiator.

Preferred photoinitiators for the visible range are α-diketones and their derivatives, such as 9,10-phenanthrenequinone, 1-phenyl-propane-1,2-dione, diacetyl or 4,4'-dichlorobenzil. Camphorquinone (CQ) and 2,2-dimethoxy-2-phenyl-acetophenone are particularly preferably used, and α-diketones in combination with amines as reducing agent, such as e.g. 4-(dimethylamino)benzoic acid ester (EDMAB), N,N-dimethylaminoethyl methacrylate, N,N-dimethyl-sym.-xylidine or triethanolamine, are quite particularly preferably used. Preferred monomolecular photoinitiators for the visible range are monoacyltrialkyl-, diacyldialkyl- and tetraacylgermanium as well as tetra-acylstannanes, such as e.g. benzoyltrimethylgermanium, dibenzoyldiethylgermani-um, bis(4-methoxybenzoyl)diethylgermanium, tetrakis(2-methylbenzoyl)germane or tetrakis(mesitoyl)stannane. Mixtures of the different photoinitiators can also be used, such as e.g. bis(4-methoxybenzoyl)diethylgermanium in combination with camphorquinone and 4-dimethylaminobenzoic acid ethyl ester.

Preferred initiators for curing the dental materials according to the invention with UV light are Norrish type I photoinitiators, above all acetophenones, e.g. 2,2-diethoxy-1-phenylethanone, benzoin ethers e.g. Irgacure 651 (benzil dimethyl ketal), hydroxyalkylphenylacetophenones, e.g. Irgacure 184 (1-hydroxycyclohexyl phenyl ketone), acyl- or bisacylphosphine oxides, e.g. Irgacure TPO (2,4,6-trimethylbenzoyldiphenylphosphine oxide), Irgacure TPO-L (ethyl phenyl(2,4,6-trimethylbenzoyl)phosphinate), TMO (2,4,6-trimethylbenzoyl)bis(p-tolyl)phosphine oxide and Irgacure 819 (bis(2,4,6-trimethylbenzoyl)phenylphosphine oxide). Further preferred photoinitiators are 2-benzyl-2-(dimethylamino)-4'-morpholinobutyrophenone (Irgacure 369) and 1-butanone-2-(dimethylamino)-2-(4-methylphenyl)methyl-1-4-(4-morpholinyl)phenyl (Irgacure 379). Particularly preferred photoinitiators are Irgacure TPO, TMO, Irgacure TPO-L and Irgacure 819. UV photoinitiators are preferred for 3D printing applications.

For a post-tempering, it is advantageous to use two photoinitiators which differ in their absorption ranges, such as e.g. Irgacure TPO and camphorquinone/4-(dimethyl-amino)benzoic acid ester.

The dental materials according to the invention can alternatively or additionally also contain thermal initiators, e.g. azo compounds, such as 2,2'-azobis(isobutyronitrile) (AIBN) or azobis-(4-cyanovaleric acid), or peroxides, such as dibenzoyl peroxide, dilauroyl peroxide, tert-butyl peroctoate, tert-butyl perbenzoate or di-(tert-butyl) peroxide. Combinations with aromatic amines can also be used to accelerate the initiation by means of peroxides. Preferred redox systems are combinations of dibenzoyl peroxide with amines, such as N,N-dimethyl-p-toluidine, N,N-dihydroxyethyl-p-toluidine, p-dimethylaminobenzoic acid ethyl ester, or structurally related systems.

The initiator or initiators are preferably used in a total quantity of from 0.1 to 5.0 wt. %, particularly preferably 0.2 to 4 wt.% and quite particularly preferably 0.3 to 3.0 wt. %, wherein these quantities include all initiator constituents, such as e.g. reducing agents.

To further improve the fracture toughness and impact resistance, the dental materials according to the invention can also contain a certain proportion of one or more core-shell polymers (**component (f)).** Core-shell polymers (CSP) with a soft polymer core, e.g. consisting of a crosslinked butyl acrylate, and a rather hard polymer shell, e.g. PMMA, are preferred. By soft or flexible polymers is meant polymers with a glass transition temperature T_{G} below 50°C, preferably below 0°C and quite particularly preferably in the range of from -30 to -110°C. PDMS, with a T_{G} of approx. -110°C, is a preferred specific example. By hard polymers is meant polymers with a glass transition temperature above 50°C and preferably above 80°C. PMMA, with a T_{G} of 100°C, is a preferred specific example.

The fracture toughness-modifying action of the CSP particles in radical di(meth)-acrylate polymer networks depends above all on the type of the CSP particles, the particle size, the crosslinking density and the weight ratio of core to shell, which preferably lies in a range of from 1:1 to 200:1. The crosslinking density is substantially determined by the proportion of crosslinking monomers in the particle core. This preferably lies in a range of from 1 to 10 wt.%, relative to the mass of the core. Particles with a particle size of from 0.20 to 5.0 µm are preferred according to the invention.

CSP particles with a core made of soft plastics, such as polybutadiene, polyisoprene, polybutyl acrylate, MMA-butadiene-styrene copolymers (MBS) or polydimethylsiloxane, and a shell made of hard plastics, such as PMMA or MMA-styrene copolymer, are preferred according to the invention. CSP particles suitable according to the invention are commercially available, e.g. from Arkema (Clearstrength), Soken (Chemisnow) or Kaneka (e.g. M521 or M210).

Core-shell polymers can be added in a quantity of up to 15 wt.%. A disadvantage of the use of core-shell polymers is that they can greatly impair the transparency of the compositions, which has a negative effect on the curing depth in the case of photopolymerization and additionally has a negative aesthetic effect in the case of dental shaped bodies. Materials which contain at most 5 wt.% and particularly preferably no core-shell particles are therefore preferred according to the invention. When incorporating the CSP particles into the dental material, a good dispersion is to be ensured.

To adjust the mechanical properties, the dental materials according to the invention can be strengthened with inorganic particulate **fillers (g).**

Preferred inorganic fillers are oxides, such as SiO₂, ZrO₂ and TiO₂ or mixed oxides of SiO₂, ZrO₂, ZnO and/or TiO₂, nanoparticulate or microfine fillers, such as fumed silica or precipitated silica, glass powders, such as quartz, glass ceramic, borosilicate or radiopaque glass powders, preferably barium or strontium aluminium silicate glasses, and radiopaque fillers, such as ytterbium trifluoride, tantalum(V) oxide, barium sulfate or mixed oxides of SiO₂ with ytterbium(III) oxide or tantalum(V) oxide. The dental materials according to the invention can furthermore contain fibrous fillers, nanofibers, whiskers or mixtures thereof.

Preferably, the oxides have a particle size of from 0.005 to 15 µm, the nanoparticulate or microfine fillers have a particle size of from 5 to 300 nm, the glass powders have a particle size of from 0.01 to 15 µm, preferably of from 0.2 to 1.5 µm, and the radiopaque fillers have a particle size of from 0.2 to 5 µm.

Particularly preferred fillers are mixed oxides of SiO₂ and ZrO₂, with a particle size of from 5 to 300 nm, glass powders with a particle size of from 0.2 to 1.5 µm, in particular radiopaque glass powders of e.g. barium or strontium aluminium silicate glasses, and radiopaque fillers with a particle size of from 0.2 to 5 µm, in particular ytterbium trifluoride and/or mixed oxides of SiO₂ with ytterbium(III) oxide.

To improve the bond between the filler particles and the crosslinked polymerization matrix, SiO₂-based fillers can be surface-modified with (meth)acrylate-functionalized silanes. A preferred example of such silanes is 3-methacryloyloxypropyltrimethoxysilane. For the surface modification of non-silicate fillers such as ZrO₂ or TiO₂, functionalized acidic phosphates, such as e.g. 10-methacryloyloxydecyl dihydrogen phosphate can also be used.

Further preferred fillers are particulate waxes, in particular carnauba wax, preferably with a particle size of from 1 to 10 µm, non-crosslinked or partially crosslinked polymethyl methacrylate (PMMA) particles, preferably with a particle size of from 500 nm to 10 µm, as well as polyamide-12 particles, preferably with a particle size of from 5 to 10 µm.

Moreover, the dental materials according to the invention can contain a so-called prepolymer filler or isofiller, i.e. a ground composite which preferably has a broad particle-size distribution, e.g. with particle sizes of from 0.05 to 20 µm, in particular approximately 0.1 to approximately 10 µm. The prepolymer filler or isofiller is preferably surface-modified, in particular silanized.

Unless otherwise stated, all particle sizes herein are weight-average particle sizes, wherein the particle-size determination in the range of from 0.1 µm to 1000 µm is effected by means of static light scattering, preferably using an LA-960 static laser scattering particle size analyzer (Horiba, Japan). Here, a laser diode with a wavelength of 655 nm and an LED with a wavelength of 405 nm are used as light sources. The use of two light sources with different wavelengths makes it possible to measure the entire particle-size distribution of a sample in only one measurement pass, wherein the measurement is carried out as a wet measurement. For this, a 0.1 to 0.5% aqueous dispersion of the filler is prepared and the scattered light thereof is measured in a flow cell. The scattered-light analysis for calculating particle size and particle-size distribution is effected in accordance with the Mie theory according to DIN/ISO 13320.

Particle sizes smaller than 0.1 µm are preferably determined by means of dynamic light scattering (DLS). The measurement of the particle size in the range of from 5 nm to 0.1 µm is preferably effected by dynamic light scattering (DLS) of aqueous particle dispersions, preferably using a Malvern Zetasizer Nano ZS (Malvern Instruments, Malvern UK) with an He-Ne laser with a wavelength of 633 nm, at a scattering angle of 90° at 25°C.

The light scattering decreases as the particle size decreases. Particle sizes smaller than 0.1 µm can also be determined by means of SEM or TEM spectroscopy. The transmission electron microscopy (TEM) is preferably carried out using a Philips CM30 TEM at an accelerating voltage of 300 kV. For the preparation of the samples, drops of the particle dispersion are applied to a 50 Å thick copper grid (mesh size 300), which is coated with carbon, and then the solvent is evaporated.

The fillers are divided according to their particle size into macrofillers and microfillers, wherein fillers with an average particle size of from 0.2 to 10 µm are called macrofillers and fillers with an average particle size of from approx. 5 to 100 nm are called microfillers. Macrofillers are obtained e.g. by grinding e.g. quartz, radiopaque glasses, borosilicates or ceramic and usually consist of splintery parts. Fumed SiO₂ or precipitated silica, or mixed oxides, e.g. SiO₂-ZrO₂, which are available by hydrolytic co-condensation of metal alkoxides, are preferably used as microfillers. The microfillers preferably have an average particle size of from approx. 5 to 100 nm. Fillers with a small particle size have a greater thickening action.

In a preferred embodiment, the dental materials according to the invention contain a mixture of two or more fillers, in particular of two or more fillers with different particle sizes. It was found that the use of such filler mixtures does not increase the viscosity of the materials excessively and the compositions can therefore be processed well using additive processes, such as e.g. using stereolithography. The total filler content preferably lies in a range of from 0 to 40 wt.%, more preferably of from 0 to 10 wt.% and most preferably 0 to 5 wt.%.

The dental materials according to the invention can furthermore contain one or more **UV absorbers (h).** By adding a UV absorber, the penetration depth of the light and thus the curing depth can be reduced, which increases the precision of the light-induced curing of the composition. This is particularly advantageous for stereolithographic applications, as only thin layers are to be cured.

UV absorbers based on benzotriazole, benzophenone or triazines are preferred. Particularly preferred UV absorbers are 2,2'-methylenebis[6-(2H-benzotriazol-2-yl)-4-(1,1,3,3-tetramethylbutyl)phenol], 2,2',4,4'-tetrahydroxybenzophenone, 2-tert-butyl-6-(5-chloro-2H-benzotriazol-2-yl)-4-methylphenol (bumetrizole), 2,2'-benzene-1,4-diyl-bis(4H-3,1-benzoxazin-4-one), 2-(4,6-bis-(2,4-dimethylphenyl)-1,3,5-triazin-2-yl)-5-(octyloxy)-phenol, 2-(2-hydroxy-5-methylphenyl)benzotriazole, 2-(2-hydroxyphenyl) benzotriazole, 2-(2H-benzotriazol-2-yl)-6-dodecyl-4-methylphenol, 2-(2'-hydroxy-3',5'-di-t-butylphenyl)-5-chlorobenzotriazole, 2,2'-dihydroxy-4-methoxybenzophenone and 2,2'-dihydroxy-4,4'-dimethoxybenzophenone. So-called Hindered Amine Light Stabilizers such as bis(1,2,2,6,6-pentamethyl-4-piperidyl) sebacate, methyl-1,2,2,6,6-pentamethyl-4-piperidyl sebacate, bis(1-octyloxy-2,2,6,6-tetramethyl-4-piperidyl) sebacate and bis(1,2,2,6,6-pentamethyl-4-piperidyl)-[[3,5-bis(1,1-dimethylethyl)-4-hydroxyphenyl]methyl]butyl malonate are further preferred. Quite particularly preferred UV absorbers are bumetrizole and 2,2',4,4'-tetrahydroxybenzophenone.

The UV absorber preferably has an absorption maximum which corresponds to the wavelength of the light used for the curing. UV absorbers with an absorption maximum in the range of from 320 to 500 nm and preferably 380 to 480 nm are advantageous, wherein UV absorbers with an absorption maximum below 400 nm are particularly preferred.

UV absorbers are optionally used in a quantity of from preferably 0 to 1.0 wt.%, particularly preferably 0.01 to 0.5 wt.%. Bumetrizole is preferably used in a quantity of from 0.01 to 0.2 wt.%, particularly preferably 0.02 to 0.15 wt.%, and 2,2',4,4'-tetrahydroxybenzophenone in a quantity of from 0.01 to 0.07 wt.%. All data relate to the total weight of the material. Dental materials which do not contain a UV absorber are preferred.

The dental materials according to the invention can also contain one or more **optical brighteners (i).** Optical brighteners differ from UV absorbers in that they are fluorescent, i.e. when exposed to radiation they emit light with a longer wavelength. Optical brighteners which absorb light in the UV range, i.e. light with a wavelength below 400 nm, are preferred according to the invention. Optical brighteners which absorb light in the UV range and emit light with a wavelength of from 400 to 450 nm are particularly preferred. Such optical brighteners increase the reactivity of the materials because, due to their fluorescence, they emit the absorbed short-wave light as longer-wave blue light and thus provide additional luminous power for the photoinitiation. Optical brighteners preferred according to the invention are 2,5-bis(5-tert-butyl-benzoxazol-2-yl)thiophene and fluorescent agents in the form of terephthalic acid derivatives, such as e.g. 2,5-dihydroxyterephthalic acid diethyl ester or diethyl-2,5-dihydroxytereph-thalate.

The optical brightener or optical brighteners are optionally used in a quantity of from preferably 0 to 0.1 wt.%, particularly preferably 0.001 to 0.05 wt.% and quite particularly preferably 0.002 to 0.02 wt.%, in each case relative to the total weight of the material. Dental materials which do not contain an optical brightener are preferred.

Optical brighteners can be used in combination with UV absorbers. In this case, it is preferred that the weight ratio of UV absorber to optical brightener lies in a range of from 2:1 to 50:1, particularly preferably 2:1 to 30:1 and quite particularly preferably 2:1 to 5:1 or 10:1 to 25:1. Combinations which contain 2,2',4,4'-tetrahydroxybenzophenone or bumetrizole as UV absorber and 2,5-bis(5-tert-butyl-benzoxazol-2-yl)-thiophene as optical brightener are preferred. The combination of 2,2',4,4'-tetrahydroxybenzophenone and 2,5-bis(5-tert-butyl-benzoxazol-2-yl)thiophene in a weight ratio of from 2:1 to 10:1, preferably 2:1 to 5:1, or the combination of bumetrizole and 2,5-bis(5-tert-butyl-benzoxazol-2-yl)thiophene in a weight ratio of from 5:1 to 30:1, preferably 10:1 to 20:1, is quite particularly preferred.

The dental materials according to the invention can additionally contain further **additives (j),** above all stabilizers, colorants, plasticizers, thixotropic additives, microbio-cidal active ingredients and/or foaming agents.

The dental materials according to the invention preferably contain one or more **stabilizers.** These are free-radical-scavenging substances for preventing a premature polyreaction. The stabilizers are also called polymerization inhibitors. The inhibitors or stabilizers improve the storage stability of the materials.

Preferred inhibitors are phenols, such as hydroquinone monomethyl ether (MEHQ) or 2,6-di-tert-butyl-4-methylphenol (BHT). Phenols are preferably used in a concentration of from 0.001 to 0.50 wt.%. Further preferred inhibitors are phenothiazine, the 2,2-diphenyl-1-picrylhydrazyl (DPPH) radical, the galvinoxyl radical, the triphenylmethyl radical and the 2,2,6,6-tetramethylpiperidine-1-oxyl (TEMPO) radical. These inhibitors are preferably used in a quantity of from 0.001 to 0.02 wt.%. A polymerization does not take place until these additives have been used up. The quantities relate in each case to the total mass of the material. A mixture of inhibitors which contains at least one phenol and at least one of the further initiators is preferably used.

In addition, the dental materials according to the invention can also contain **colorants,** preferably in a concentration of from 0.0001 to 0.5 wt.%. The colorants are primarily used for aesthetic purposes. Colorants preferred according to the invention are organic dyes and pigments, in particular azo dyes, carbonyl dyes, cyanine dyes, azomethines and methines, phthalocyanines and dioxazines. Dyes which are soluble in the materials according to the invention, in particular azo dyes, are particularly preferred. Moreover, inorganic and in particular organic pigments which can be dispersed well in the dental materials according to the invention are suitable as colorant. Preferred inorganic pigments are metal oxides or hydroxides, such as e.g. titanium dioxide or ZnO as white pigments, iron oxide (Fe₂O₃) as red pigment or iron hydroxide (FeOOH) as yellow pigment. Preferred organic pigments are azo pigments, such as e.g. monoazo yellow and orange pigments, diazo pigments or β-naphthol pigments, and non-azo or polycyclic pigments, such as e.g. phthalocyanine, quinacridone, perylene and flavanthrone pigments. Azo pigments and non-azo pigments are particularly preferred.

Moreover, the dental materials according to the invention can contain one or more **plasticizers.** Plasticizers prevent the polymers from becoming brittle after the photochemical curing and possible drying. In addition, plasticizers ensure sufficient flexibility. Plasticizers are preferably added in a concentration of from 0.2 to 5 wt.%. Preferred plasticizers are phthalates, such as e.g. dibutyl or dihexyl phthalate, non-acidic phosphates, such as e.g. tributyl or tricresyl phosphate, n-octanol, glycerol or polyethylene glycols. Tartaric acid ester or citric acid ester, such as e.g. citric acid triester, which are characterized by a good biocompatibility, are particularly preferred.

The dental materials according to the invention can furthermore contain one or more **thixotropic additives.** These additives bring about a thickening of the materials and can thus, for example, prevent the fillers from sedimenting. In particular, filler-containing materials therefore preferably contain at least one thixotropic additive. Preferred thixotropic additives are OH group-containing polymers, such as e.g. cellulose derivatives, and inorganic substances, such as e.g. layer silicates. In order not to increase the viscosity of the materials too much, the dental materials according to the invention preferably contain only 0 to 3.0 wt.%, particularly preferably 0 to 2.0 wt.% and quite particularly preferably 0.1 to 2.0 wt.% thixotropic additive, relative to the total weight of the material.

Certain fillers, such as e.g. highly dispersed SiO₂, i.e. SiO₂ with a small primary particle size (< 20 nm) and a large surface area (> 100 m²), likewise have a thixotropic effect. Such fillers can replace thixotropic additives.

The rheological properties of the dental materials according to the invention are matched to the desired intended application. Materials for stereolithographic processing are preferably adjusted such that their viscosity lies in the range of from 50 mPa·s to 100 Pa·s, preferably 100 mPa·s to 10 Pa·s, particularly preferably 100 mPa·s to 5 Pa·s. The dental materials according to the invention particularly preferably have a viscosity < 10 Pa·s and quite particularly preferably < 5 Pa·s at 25°C. The viscosity is determined at 25°C using a cone-plate viscometer (shear rate 100/s), preferably with an Anton Paar MCR 302-type viscometer with a CP25-2 cone-plate measuring system and a measuring gap of 53 µm in rotation at a shear rate of 100/s. Because of the low viscosity, the dental materials according to the invention are particularly suitable for being processed using additive manufacturing processes, such as e.g. 3D printing or stereolithography. The processing temperature preferably lies in a range of from 10 to 70°C, more preferably 20 to 60°C and most preferably 20 to 50°C.

According to the invention, dental materials with the following composition are particularly preferred:
(a) 15 to 75 wt. %, preferably 20 to 70 wt.%, particularly preferably 25 to 65 wt.% of at least one mono(meth)acrylate (a),
(b) 15 to 75 wt.%, preferably 20 to 70 wt.% and particularly preferably 25 to 65 wt.% of at least one urethane di(meth)acrylate macromonomer of Formula I**,** wherein m is a value of from 0.6 to 2.0,
(c) 0 to 30 wt.%, preferably 0 to 20 wt.% and particularly preferably 0 to 10 wt.% of one or more di(meth)acrylate monomers,
(d) 0.1 to 10 wt.%, especially 2 to 10 wt.%, preferably 3 to 9 wt.%, particularly preferably 4 to 8 wt.% of at least one (PCL)_{q}-b-(PDMS)ᵣ-b-(PCL)_{q} block copolymer having a number-average molar mass of 2000 to 10000 g/mol,
(e) 0.1 to 3.0 wt.%, preferably 0.3 to 2.5 wt.% and particularly preferably 0.5 to 2.0 wt.% of at least one initiator for the radical polymerization,
(f) 0 to 15 wt. %, preferably 0 to 5 wt.% and particularly preferably 0 wt.% of core-shell polymer particles,
(g) 0 to 20 wt.%, preferably 0 to 15 wt.% and particularly preferably 0 to 10 wt.% of filler,
(h) 0 to 1.0 wt.%, preferably 0 to 0.7 wt.% and particularly preferably 0 to 0.5 wt.% of UV absorber,
(i) 0 to 0.5 wt.%, preferably 0 to 0.1 wt.% and particularly preferably 0 to 0.05 wt.% of optical brightener and
(j) 0 to 15 wt. %, preferably 0 to 10 wt.% and particularly preferably 0.05 to 5 wt.% of further additives.

Dental materials with the following composition are quite particularly preferred:
(a) 15 to 75 wt.%, preferably 20 to 70 wt.%, particularly preferably 25 to 65 wt.% of (octahydro-4,7-methano-1H-indenyl)methyl acrylate (OMIMA), isobornyl acrylate (IBOA) or a mixture thereof,
(b) 15 to 75 wt.%, preferably 20 to 70 wt.% and particularly preferably 25 to 65 wt.% of at least one urethane di(meth)acrylate macromonomer of Formula I, wherein *m* is a value of from 0.7 to 1.6,
(c) 0 to 30 wt.%, preferably 0 to 20 wt.% and particularly preferably 0 to 10 wt.% of di(meth)acrylate monomer(s),
(d) 2 to 10 wt.%, preferably 3 to 9 wt.%, particularly preferably 4 to 8 wt.% of at least one (PCL)_{q}-b-(PDMS)ᵣ-b-(PCL)_{q} block copolymer having a number-average molar mass of 2000 to 10000 g/mol,
(e) 0.1 to 3.0 wt. %, preferably 0.3 to 2.5 wt.% and particularly preferably 0.5 to 2.0 wt.% of at least one initiator for the radical polymerization,
(f) 0 to 15 wt.%, preferably 0 to 5 wt.% and particularly preferably 0 wt.% of core-shell polymer particles,
(g) 0 to 20 wt.%, preferably 0 to 15 wt.% and particularly preferably 0 to 10 wt.% of filler,
(h) 0 to 1.0 wt.%, preferably 0 to 0.7 wt.% and particularly preferably 0 to 0.5 wt.% of UV absorber,
(i) 0 to 0.5 wt.%, preferably 0 to 0.1 wt.% and particularly preferably 0 to 0.05 wt.% of optical brightener and
(j) 0 to 15 wt.%, preferably 0 to 10 wt.% and particularly preferably 0.05 to 5 wt.% of further additives.

Unless otherwise stated, all percentages by weight herein relate to the total mass of the dental material.

Dental materials which contain
(a) 20 to 70 wt.% of (octahydro-4,7-methano-1H-indenyl)methyl acrylate (OMIMA), isobornyl acrylate (IBOA) or a mixture thereof,
(b) 20 to 70 wt.% of at least one urethane di(meth)acrylate macromonomer of Formula I with at least 4 urethane groups, prepared by reacting 1 eq. tricy-clo[5.2.1.0^{2,6}]decanedimethanol with 2 eq. TMDI and then reacting with 2 eq. 2-hydroxyethyl methacrylate (HEMA) or hydroxypropyl methacrylate (HPMA), wherein m is a value of from 0.9 to 1.6,
(c) 0 wt.% of further di(meth)acrylate monomers,
(d) 3 to 9 wt.% of at least one PCL)_{q}-b-(PDMS)ᵣ-b-(PCL)_{q} block copolymer having a number-average molar mass of 2000 to 10 000 g/mol,
(e) 0.3 to 2.5 wt.% of at least one photoinitiator,
(f) 0 wt.% of core-shell polymer particles,
(g) 0 to 5 wt.% of one or more fillers, preferably fumed silica, and
   0.2 to 5 wt.% of one or more UV absorbers, optical brighteners and/or further additives,
are particularly preferred according to the invention.

The dental materials according to the invention are characterized in that they have a high fracture toughness and fracture work and at the same time a good flexural strength and a relatively high modulus of elasticity, measured at 37°C in water, which corresponds to oral conditions. The materials also have a high transparency and a low viscosity. It is particularly advantageous that the dental materials still have a high transparency and a low intrinsic color even after curing.

According to the invention, materials with a transparency ≥ 60%, preferably ≥ 70% and quite particularly preferably ≥ 80%, and a viscosity ≤ 10.0 Pa·s, preferably ≤ 5,0 Pa·s, are particularly preferred. The transparency is measured in accordance with the ISO 10526:1999 standard, as described above. The viscosity is determined using a cone-plate viscometer in the manner described above.

After curing, the materials according to the invention preferably have a maximum stress intensity factor Kₘₐₓ of greater than 1.5 MPa·m^{1/2}, preferably greater than 1.7 MPa·m^{1/2}, particularly preferably greater than 1.9 MPa·m^{1/2} as well as a total fracture work Wf greater than 600 J/m², preferably greater than 800 J/m², particularly preferably greater than 900 J/m². Workpieces which are produced from these materials thus withstand, to a high degree, deformations without fracturing. A high transparency in combination with a high fracture work cannot be achieved with core-shell polymers. Kₘₐₓ and Wf can be adjusted to the desired values by varying the amount of the block copolymer (d) as well as the ratio of the macromonomer to the block copolymer.

The determination of the maximum stress intensity factor Kₘₐₓ and the total fracture work Wf is performed in accordance with ISO 20795-1:2013 in the 3-point flexural test with a support span of 32 mm. The determination of Kₘₐₓ and Wf is based on the theoretical principles of the stress intensity factor K_{1C}. The maximum stress intensity factor Kₘₐₓ (in MPa·m^{1/2}) is the highest factor of the stress intensity, which is also called the stress intensity factor at highest load, and is calculated as follows: ${K}_{max} = \frac{f \left(x\right) {P}_{max} {l}_{t}}{\left({b}_{t}{h}_{t}^{\frac{3}{2}}\right)} \times \sqrt{{10}^{- 3}} MPa {m}^{\frac{1}{2}}$ where f(x) is a geometrical function dependent on x according to the following formula: $f \left(x\right) = 3 {x}^{\frac{1}{2}} \left[1.99-x\left(1-x\right)\left(2.15-3.93x+2.7{x}^{2}\right.\right] / \left[2\left(1+2x\right){\left(1-x\right)}^{\frac{3}{2}}\right]$ wherein x = (a / ht), *hₜ* is the height of the specimen (8 mm), *bₜ* is its width (4 mm), *lₜ* is the span length (32 mm), a is the crack length (3 mm + crack depth with razor blade), and *Pₘₐₓ* is the maximum load exerted on the specimens (in N).

The total fracture work *W_{f}* (in J/m²) is calculated as follows: ${W}_{f} = \frac{U}{\left[2{b}_{t}\left({h}_{t}-a\right)\right]} \times 1000$ where U is the recorded area under the load/deflection curve. W_{f} describes the resistance of the material to crack propagation.

After curing, the materials according to the invention also have a good flexural strength and a relatively good flexural modulus. Shaped parts which are obtained by curing the materials according to the invention have a high stiffness and oppose a deformation with a high level of resistance without fracturing. Materials which have a flexural strength after curing, determined in accordance with ISO20795-1:2013, of at least 40 MPa, particularly preferably 50 MPa or more and quite particularly preferably of 60 MPa or more are preferred. Moreover, the cured materials preferably have a flexural modulus, determined in accordance with ISO20795-1:2013, of at least 1000 MPa, preferably of 1300 MPa or more, particularly preferably of 1500 MPa or more, quite particularly preferably 2000 MPa.

Due to the above properties, the materials according to the invention are excellently suited for use as a dental material, e.g. as a prosthetic material or veneering material, and in particular for the production or repair of dental shaped parts, such as e.g. dental restorations, prostheses, artificial teeth, inlays, onlays, crowns, bridges, drilling templates, splints (bite splints), try-in bodies and orthodontic appliances, such as e.g. plastic correction splints, so-called aligners and positioners. The named shaped parts are also a subject of the invention. The dental materials according to the invention are preferably used extraorally, i.e. non-therapeutically.

A further subject of the present invention is a process for the production of dental shaped parts, in particular for the production of the above-named dental shaped parts, in which a composition according to the invention is cured with the aid of light in order to give the dental shaped part. The production or repair of dental shaped parts is preferably effected extraorally, particularly preferably by an additive process, quite particularly preferably by 3D printing or a lithography-based process, such as e.g. stereolithography.

The stereolithographic production of shaped parts is preferably effected by creating a virtual image of the tooth situation by direct or indirect digitization of the tooth to be restored or of the teeth to be restored on a computer, then constructing a model of the dental restoration or prosthesis on the computer on the basis of this image and subsequently producing this model by additive stereolithographic manufacturing.

Once the virtual model of the dental workpiece to be produced has been created, the composition according to the invention is polymerized by selective light irradiation. The dental restoration or prosthesis is preferably constructed in layers by polymerizing a plurality of thin layers with the desired cross section one after another. In stereolithography (SL), a UV laser is preferably used as the light source. The layers are preferably cured by digital light processing (DLP). In this process, a projectable image is used for curing the photopolymerization resin in layers. Alternatively, this image may be projected directly from an LCD or LED screen.

After the layered construction of the restoration or prosthesis, excess residual resin is preferably removed. This can be effected by suitable mechanical processes (e.g. centrifuging or sandblasting) or by treatment with a suitable solvent, e.g. an alcohol, such as ethanol or isopropanol, a ketone, such as e.g. acetone, or an ester, such as e.g. ethyl acetate. A post-tempering is then preferably effected by heating or particularly preferably by irradiation of the workpiece with light of a suitable wavelength, such as e.g. irradiation with light with an intensity of e.g. 160 mW/cm² at 405 nm. When two photoinitiators are used, irradiation with two different wavelengths is advantageous. The workpiece is preferably heated to a temperature above 50°C at the same time or in a subsequent step. The mechanical properties can be improved through the photochemical and/or thermal post-tempering.

The invention is explained in more detail in the following with reference to examples.

### Examples

### Example 1

### Synthesis of the urethane di(meth)acrylate macromonomer DMA1 (m = 1.0)

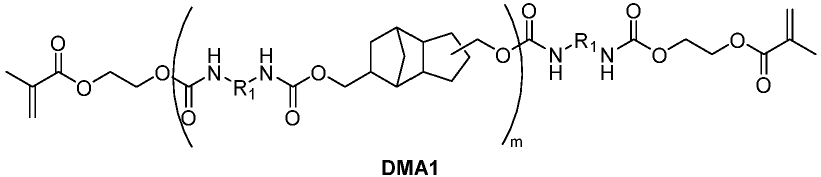

wherein R¹ is an isomer mixture of the following alkyl chains: R¹

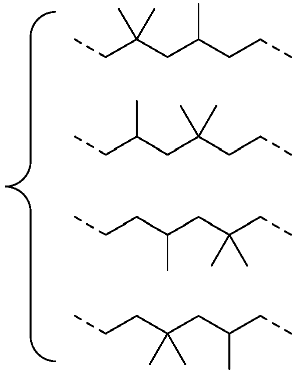

A mixture of tricyclo[5.2.1.0^{2,6}]decanedimethanol (223.77 g, 1.14 mol) and dibutyltin dilaurate (0.63 g, 1.0 mmol) was heated to 40 °C. TMDI (479.44 g, 2.28 mol) was added and the reaction mixture was stirred at 100 °C for 1 h. Subsequently, BHT (0.22 g, 1.0 mmol) and HEMA (304.55 g, 2.34 mol) were added, and the resulting mixture was stirred for 1 h, affording 988.00 g of the desired product.

Yield: 99 %. Aspect: colorless resin.

¹H NMR (CDCI₃, 400 MHz): δ = 0.83-0.98 (m, 18H; CH₃); 1.00-1.83 (m, 18H; CH, CH₂); 1.95 (s, 6H; CH₃); 1.99-2.58 (m, 6H; CH, CH₂); 2.78-3.26 (m, 8H; NCH₂); 3.74-4.00 (m, 4H; OCH₂); 4.25-4.45 (m, 8H; OCH₂); 4.51-5.29 (m, 4H; NH); 5.58 (s, 2H; =CH); 6.14 (s, 2H; =CH).

### Example 2

### Synthesis of urethane di(meth)acrylate macromonomer DMA2 (m = 1.0)

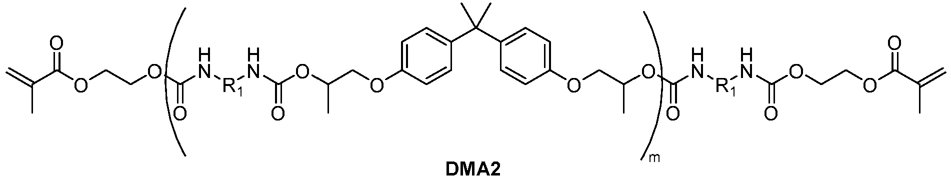

wherein R¹ is an isomer mixture of the following alkyl chains: R¹

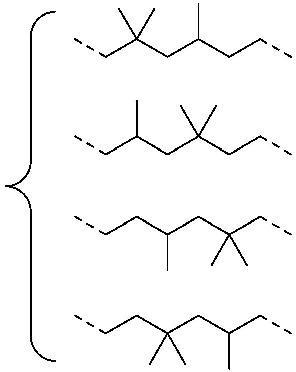

A mixture of TMDI (210.28 g, 1.00 mol, 2.0 equiv.) and dibutyltin dilaurate (0.30 g, 0.5 mmol) was heated to 40 °C. 1,1'-Isopropylidenebis(p-phenyleneoxy)dipropan-2-ol (172.23 g, 0.50 mol, 1.0 equiv.) was added and the reaction mixture was stirred at 100 °C for 1 h. Subsequently, BHT (0.13 g, 0.6 mmol) and HEMA (132.09 g, 1.02 mol, 2.03 equiv.) were added, and the resulting mixture was stirred for 1 h, affording 484.47 g of the desired product.

Yield: 95 %. Aspect: colorless resin.

¹H NMR (CDCI₃, 400 MHz): δ = 0.79-0.99 (m, 18H, CH₃); 1.00-1.76 (m, 22H, CH, CH₂, CH₃); 1.95 (s, 6H, CH₃); 2.75-3.29 (m, 8H, NCH₂); 3.73-4.05 (m, 4H, OCH₂); 4.08-4.43 (m, 8H, OCH₂); 4.46-5.24 (m, 6H, NH, OCH); 5.58 (s, 2H, =CH); 6.13 (s, 2H, =CH); 6.70-6.86 (m, 4H, Ar-H); 7.03-7.17 (m, 4H, Ar-H).

### Example 3

### Synthesis of urethane di(meth)acrylate macromonomer DMA3 (m = 1.0)

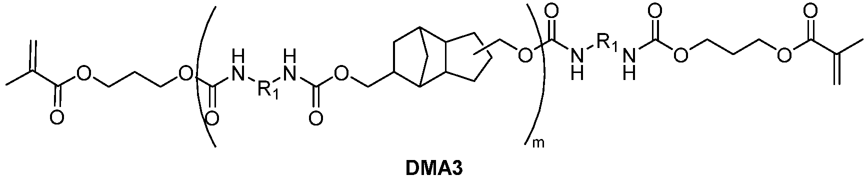

wherein R¹ is an isomer mixture of the following alkyl chains: R¹

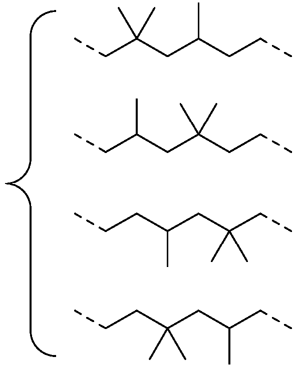

A mixture of TMDI (51.10 g, 0.24 mol, 2.00 equiv.) and dibutyltin dilaurate (0.64 g, 0.1 mmol) was heated to 40 °C. tricyclo[5.2.1.0^{2,6}]decanedimethanol (23.85 g, 0.12 mol, 1.00 equiv.) was added and the reaction mixture was stirred at 100 °C for 1 h. Subsequently, BHT (0.51 g, 0.23 mmol) and 3-hydroxypropyl methacrylate (35.91 g, 0.25 mol, 2.05 equiv.) were added and the resulting mixture was stirred for 1 h, affording 102.36 g of the desired product.

Yield: 93 %. Aspect: colorless resin.

¹H NMR (CDCI₃, 400 MHz): δ = 0.82-0.99 (m, 18H, CH₃); 1.00-1.84 (m, 18H, CH, CH₂); 1.95 (s, 6H, CH₃); 1.99-2.58 (m, 10H, CH, CH₂); 2.77-3.29 (m, 8H, NCH₂); 3.67-4.00 (m, 4H, OCH₂); 4.08-4.35 (m, 8H, OCH₂); 4.53-5.22 (m, 4H, NH); 5.57 (s, 2H, =CH); 6.11 (s, 2H, =CH).

### Example 4 (comparative)

### Synthesis of urethane dimethacrylate macromonomer DMA4 (m = 1.0)

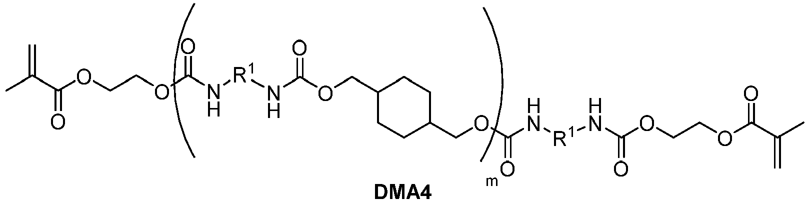

wherein R¹ is an isomer mixture of the following alkyl chains: R¹

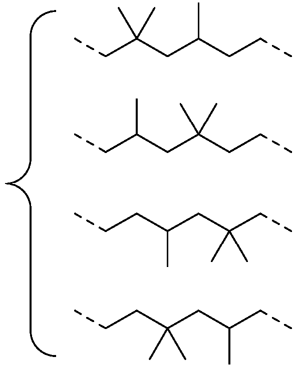

A mixture of TMDI (223.33 g, 1.11 mol, 2.00 equiv.) and dibutyltin dilaurate (0.29 g, 0.5 mmol) was heated to 40 °C. 1,4-Cyclohexanedimethanol (80.00 g, 0.56 mol, 1.00 equiv.) was added and the reaction mixture was stirred at 100 °C for 1 h. Subsequently, BHT (0.15 g, 0.6 mmol) and 3-hydroxypropyl methacrylate (148.00 g, 1.14 mol, 2.05 equiv.) were added and the resulting mixture was stirred for 1 h, affording 426.7 g of the desired product as a colorless resin.

Yield: 93 %.

¹H NMR (CDCI₃, 400 MHz): δ = 0.82-0.97 (m, 18H, CH₃); 1.00-1.90 (m, 20H, CH, CH₂); 1.95 (s, 6H, CH₃); 2.79-3.27 (m, 8H, NCH₂); 3.82-4.05 (m, 4H, OCH₂); 4.25-4.42 (m, 8H, OCH₂); 4.66-5.19 (m, 4H, NH); 5.56-5.62 (m, 2H, =CH); 6.11-6.17 (m, 2H, =CH).

### Example 5 (comparative)

### Synthesis of urethane dimethacrylate macromonomer DMA5 (m = 1.0)

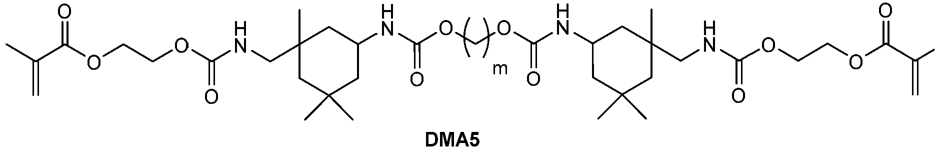

A mixture of 1,6-hexanediol (177.3 g, 1.50 mol), isophorone diisocyanate (666.9 g, 3.00 mol) and a catalytic amount of dibutyltin dilaurate (DBTDL) (0.45 g, 0.71 mmol) was heated up to 100 °C. After 1 h of reaction, the temperature was reduced to 90 °C and BHT (0.37 g, 1.68 mmol) and 3-hydroxypropyl methacrylate (390.4 g, 3.00 mol, 2.00 equiv.) were added. The resulting mixture was stirred for 1 h, affording 1115.7 g of the desired product as a colorless resin.

Yield: 90 %.

¹H NMR (CDCl₃, 400 MHz): δ = 0.80-1.14, 1.14-1.28, 1.31-1.46 and 1.52-1.81 (4 m, 38H, CH₂ and CH₃); 1.95 (s, 6H, CH₃); 2.79-3.02 and 3.09-3.46 (2 m, 4H, CH₂NHCOO); 3.51-3.85 (m, 2H, CHNHCOO); 3.95-4.15 (m, 4H, CH₂OCO); 4.21-4.42 (m, 8H, CH₂OCO); 4.45-5.00 (m, 4H, NHCOO); 5.60 (s, 2H, =C₂); 6.14 (s, 2H, =CH).

### Example 6 (comparative)

### Synthesis of urethane dimethacrylate macromonomer DMA6

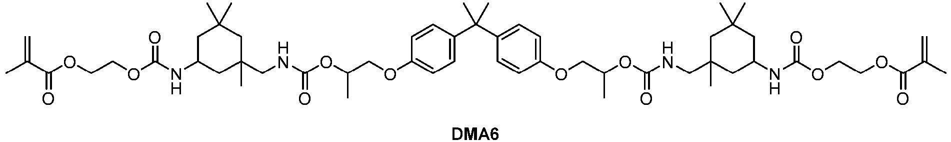

1,1'-Isopropylidenebis(p-phenyleneoxy)dipropan-2-ol (15.08 g, 43.78 mmol) and dibutyltin dilaurate (DBTDL, 91.7 mg) were dissolved in DCM (50 mL). The resulting solution was heated up to 40 °C. IPDI (19.47 g, 87.59 mmol, 2.00 equiv.) was added dropwise. The reaction mixture was stirred at 40 °C for 2 h. HEMA (11.68 g, 89.75 mmol, 2.05 equiv.) was added dropwise and the reaction mixture was stirred at 40 °C for 3 h. The solvent was removed under reduced pressure, affording 45.9 g of the desired product as a colorless resin.

Yield: quantitative.

¹H NMR (400 MHz, CDCl₃): δ = 0.75-0.95 (m, 12H, CH₃), 0.95-1.10 (m, 12H, CH₂,CH₃), 1.10-1.25 (m, 2H, CH₂), 1.25-1.45 (m, 6H, CH₃), 1.62 (s, 6H, CH₃), 1.63-1.72 (m, 4H, CH₂), 1.96 (s, 6H, CH₃), 2.92 (bs, 4H, NCH₂), 3.70-3.90 (m, 2H, NCH), 3.90-4.05 (m, 4H, CH₂OAr), 4.35-4.45 (m, 8H, CH₂OOCN, CH₂OOC), 4.52-4.62 (m, 2H, NH), 4.80-4.90 (m, 2H, NH), 5.05-5.15 (m, 2H, CHOOC), 5.59 (s, 2H, C CH₂), 6.15 (s, 2H, C CH₂), 6.80 (d, J = 8 Hz, 4 H, ArH), 7.12 (d, J = 8 Hz, 4 H, ArH).

### Example 7

### Synthesis of PCL(1000)-b-PDMS(2000)-b-PCL(1000) block copolymer (BCP1)

### 1^{st} stage: tetramethylammonium 3-aminopropyl dimethylsilanoate

In a protective gas atmosphere, a mixture of 1,3-bis(3-aminopropyl)tetramethyldisiloxane (2.49 g, 10.0 mmol) and tetramethylammonium hydroxide pentahydrate (3.62 g, 20 mmol) in tetrahydrofuran (THF; 10 ml) was heated at reflux for 3 h. The solvent was distilled off and the residue was heated to 50°C in a fine vacuum. The yellowish residue was recrystallized from THF (20 ml). 3.17 g (15.4 mmol; 77%) of a white solid was obtained.

¹H-NMR (CDCI₃, 400 MHz): δ = 3.16 (s, 12H; N⁺-CH₃), 2.37 (t, 2H; J = 7.1 Hz; N-CH₂), 1.28 (m, 2H; CH₂), 0.14 (m, 2H; Si-CH₂), -0.33 (s, 6H; Si-CH₃).

### 2^{nd} stage: polydimethylsiloxane-αω-dipropyl-3-amine: PDMS(2000) (Mw=2000 g/mol)

In a protective gas atmosphere, a mixture of 1,3-bis(3-aminopropyl)-tetramethyl dis-iloxane (9.94 g, 40.0 mmol) and octamethyl cyclotetrasiloxane (12.00 g, 40 mmol) was heated to 80 °C. Tetramethylammonium-(3-aminopropyl)-dimethylsilanoate (20.6 mg, 0.10 mmol) was added and stirring was continued at 80 °C. After 30 min, octamethyl cyclotetrasiloxane saturated with argon (56.00 g, 0.192 mol) was slowly added dropwise over a period of 2 h. The reaction mixture was stirred at 80 °C for 22 h. Volatile components were then removed at 150 °C under high vacuum. 69.43 g (84 %) of a colorless oil was obtained.

¹H-NMR (CDCI₃, 400 MHz): δ = 0.15 (s, 150H; Si-CH₃); 0.58-0.65 (m, 4H; Si-CH₂); 1.27 (br s, 4H; NH₂); 1.49- 1.59 (m, 4H; CH₂); 2.75 (t, 4H; J = 7.0 Hz; N-CH₂).

### 3^{rd} stage: PCL(1000)-b-PDMS(2000)-b-PCL(1000) (BCP1; q ~ 9; r ~ 27)

A mixture of PDMS(2000) (23.60 g) and ε-caprolactone (24.00 g, 0.21 mol) was heated to 80 °C. After 0.5 h, tin(II)-bis-(2-ethylhexanoate) (20 mg, 0.05 mmol) was added and the temperature was gradually raised to 130 °C in increments of 10 °C over a period of 0.5 h. After stirring at 130 °C for 22 h, volatiles were removed under reduced pressure (0.3 mbar, 130 °C - 160 °C), yielding 46.33 g (97 %) of the block copolymer as a waxy, slightly yellowish solid.

¹H-NMR (CDCI₃, 400 MHz): δ = 0.15 (s, 150H; Si-CH₃); 0.56-0.66 (m, 4H; Si-CH₂); 1.30-1.54 (m, 35H; CH₂); 1.65-1.81 (m, 70H; CH₂); 2.25 (t, 4H; J = 7.5 Hz; N-CH₂); 2.39 (t, 31H; J = 7.5 Hz; C(O)-CH₂); 3.30 (q, 4H; J = 6.7 Hz; N-CH₂); 3.68-3.76 (m, 4H; HO-CH₂); 4.14 (t, 31H; J = 6.7 Hz; O-CH₂).

### Example 8

### Synthesis of PCL(2000)-b-PDMS(2000)-b-PCL(2000) (BCP2; g ~ 18; r ~ 27)

A mixture of PDMS(2000) (7.40 g; Example 7) and ε-caprolactone (15.13 g, 132.6 mmol) was heated to 80 °C. After 0.5 h, tin(II)-bis-(2-ethylhexanoate) (20 mg, 0.05 mmol) was added and the temperature was gradually raised to 130 °C in increments of 10 °C over a period of 0.5 h. After stirring at 130 °C for 22 h, volatiles were removed under reduced pressure (0.3 mbar, 130 °C - 160 °C), yielding 20.42 g (91 %) of the block copolymer as a waxy, slightly yellowish solid.

¹H NMR (CDCI₃, 400 MHz): δ = 0.15 (s, 150H; Si-CH₃); 0.55-0.68 (m, 4H; Si-CH₂); 1.35-1.54 (m, 70H; CH₂); 1.65-1.83 (m, 140H; CH₂); 2.24 (t, 4H; J = 7.5 Hz; N-CH₂); 2.39 (t, 66H; J = 7.5 Hz; C(O)-CH₂); 3.30 (q, 4H; J = 6.8 Hz; N-CH₂); 3.68-3.77 (m, 4H; HO-CH₂); 4.14 (t, 66H; J = 6.8 Hz; O-CH₂).

### Example 9

### Preparation and polymerization of dental resins

Dental resins as specified in Table 1 were prepared by mixing the urethane di(meth)acrylate macromonomer with the selected monofunctional monomer. Next, 1.0 wt.% TPO as well as the selected BCP were added. The mixture was stirred at 50 °C until the BCP was completely solubilized.

### Measurement of flexural strength FS and flexural modulus FM

Flexural strength was measured according to ISO20795-1:2013. Specimens (3.3 mm x 10 mm x 64 mm) were prepared using stainless-steel molds (n = 5). The molds were filled with the photopolymerizable dental resin mixture and covered with polyester film (50 µm) to avoid oxygen inhibition. After light-curing for 10 min from both sides in a PrograPrint Cure LED curing unit (120 mW/cm² @ 405 nm and 100 mW/cm² @ 465 nm), the specimens were removed from the mold and stored in water at 37 °C for 50 h. Measurement of flexural strength and modulus was carried out in a three-point bending test (span: 50 mm) with a cross-head speed of 5 mm min⁻¹ using a Z2.5/TS universal testing machine (Zwick Roell, Ulm, Germany). The results are shown in Table 1.

### Measurement of fracture toughness

Fracture toughness was measured using a modified bending test described in ISO 20795-1:2013. Both the maximum stress intensity factor (*Kₘₐₓ*) and total fracture work (*W_{f}*) were determined using single-edge notched beam (SENB) specimens. A stainless-steel mold (4 mm × 8 mm × 40 mm) was filled with a photopolymerizable dental resin mixture. The mold was covered with a polyester film (50 µm) to avoid oxygen inhibition. After light-curing for 10 min from both sides in a PrograPrint Cure LED curing unit (120 mW/cm² @ 405 nm and 100 mW/cm² @ 465 nm), cured specimens were removed from the mold and a 3.0 mm deep notch was prepared using a circular saw with diamond blade and the initial crack was prepared by striking a razorblade with gentle pressure to a depth of 0.3 mm. Specimens were stored in water for 24 h at 37 °C, dabbed with paper and subsequently loaded to break at RT with a span of 32 mm and at a crosshead speed of 1.0 mm/min using a universal testing machine (Zwick Z2.5, Zwick/Roell, Ulm, Germany).

Kₘₐₓ (in MPa*m^{1/2}) was calculated as described above using the following formula: ${K}_{max} = \frac{f \left(x\right) {P}_{max} {l}_{t}}{\left({b}_{t}{h}_{t}^{\frac{3}{2}}\right)} \times \sqrt{{10}^{- 3}} MPa {m}^{\frac{1}{2}}$

The total fracture work *W_{f}* (in J/m²) was calculated as described above using the following formula: ${W}_{f} = \frac{U}{\left[2{b}_{t}\left({h}_{t}-a\right)\right]} \times 1000$ where *U* is the recorded area under the load/deflection curve. The results are shown in Table 1.

The results show that the addition of DMA1, DMA2 or DMA3 to a mixture of BCP1 and OMIMA led to high Kmax and Wf values, combined with excellent mechanical properties (high FS and FM values). Tests Nos. 9 and 10 show that the addition of DMA1 to BCP2/OMIMA or BCP3/OMIMA also gave excellent results for Kmax and Wf. Test No. 11 shows that the addition of DMA1 to a mixture of BCP1 and IBOA gave similar results. On the other hand, the addition of DMA4, DMA5 or DMA6 to BCP1/OMIMA only provided moderate Wf values. DMAs 4 to 6 are not according to Formula 1 and values are given as comparative examples. These results clearly highlight the importance of the nature of the urethane di(meth)acrylate macromonomer structure on the toughening efficiency. In particular, DMAs 1-3 yielded significantly higher Wf values than DMAs 4-6 with the same amount of BCP1.

**Table 1: Composition and properties of dental resins**

| **Formulation** | **Urethane macromonomer / Monomer** [added to get 100 wt.%] | **Blockcopolymer (BCP)** | | **Kmax¹⁾** [MPa·m^{1/2}] | **Wf²⁾** [J/m²] | **FS³⁾** [MPa] | **FM⁴⁾** [MPa] |
|---|---|---|---|---|---|---|---|
| | | Type | [wt.%] | | | | |
| No. 1 | DMA1 / OMIMA⁵⁾: 5/5 (wt.%/wt.%) | BCP1 | 4 | 2.285 ± 0.123 | 918 ± 167 | 70.1 ± 2.0 | 2171 ± 56 |
| No. 2 | DMA1 / OMIMA⁵⁾: 5/5 (wt.%/wt.%) | BCP1 | 5 | 2.477 ± 0.142 | 1478 ± 51 | 66.0 ± 1.1 | 2037 ± 60 |
| No. 3 | DMA1 / OMIMA⁵⁾: 5/5 (wt.%/wt.%) | BCP1 | 6 | 2.277 ± 0.092 | 1567 ± 66 | 62.8 ± 0.7 | 2019 ± 36 |
| No. 4 | DMA2 / OMIMA⁵⁾: 5/5 (wt.%/wt.%) | BCP1 | 5 | 2.303 ± 0.124 | 1695 ± 43 | 62.1 ± 3.8 | 1872 ± 140 |
| No. 5 | DMA3 / OMIMA⁵⁾: 5/5 (wt.%/wt.%) | BCP1 | 5 | 2.296 ± 0.074 | 1505 ± 42 | 64.1 ± 1.1 | 2008 ± 19 |
| No. 6*) | DMA4 / OMIMA⁵⁾: 5/5 (wt.%/wt.%) | BCP1 | 5 | 2.005 ± 0.081 | 818 ± 248 | 63.5 ± 1.6 | 1911 ± 44 |
| No. 7*) | DMA5 / OMIMA⁵⁾: 5/5 (wt.%/wt.%) | BCP1 | 5 | 2.157 ± 0.074 | 705 ± 29 | n.d. | n.d. |
| No. 8*) | DMA6 / OMIMA⁵⁾: 5/5 (wt.%/wt.%) | BCP1 | 5 | 1.825 ± 0.032 | 393 ± 14 | n.d. | n.d. |
| No. 9 | DMA1 / OMIMA⁵⁾: 5/5 (wt.%/wt.%) | BCP2 | 5 | 2.447 ± 0.070 | 1704 ± 36 | 58.4 ± 2.4 | 1923 ± 98 |
| No. 10 | DMA1 / OMIMA⁵⁾: 5/5 (wt.%/wt.%) | BCP3⁷⁾ | 5 | 2.135 ± 0.038 | 1541 ± 41 | 62.9 ± 1.1 | 2017 ± 110 |
| No. 11 | DMA1 / IBOA⁶⁾: 5/5 (wt.%/wt.%) | BCP1 | 6 | 1.852 ± 0.027 | 1208 ± 41 | 63.5 ± 4.7 | 2011 ± 278 |

| | | | | | | | |
|---|---|---|---|---|---|---|---|
| *) comparison example n.d. not determined ¹⁾ maximum stress intensity factor Kmax measured in accordance with ISO 20795-1:2013 ²⁾ total fracture work Wf measured in accordance with ISO 20795-1:2013 ³⁾ flexural strength (FS) measured in accordance with ISO20795:2013 ⁴⁾ flexural modulus (FM) measured in accordance with ISO20795:2013 ⁵⁾ (octahydro-4,7-methano-1H-indenyl)methyl acrylate ⁶⁾ isobornyl acrylate ⁷⁾ PCL-b-PDMS-b-PCL block copolymer according to Example 3 of EP 4 085 893 A1, the molar mass of the PDMS blocks is 3200 g/mol and the molar mass of the PCL blocks is 1600 g/mol (q *~* 14, r *~* 43) | | | | | | | |

## Claims

1. An urethane di(meth)acrylate macromonomer of Formula I: 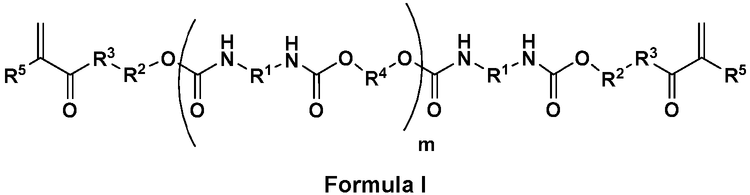 **characterized in that** the variables have the following meanings:
R¹ is 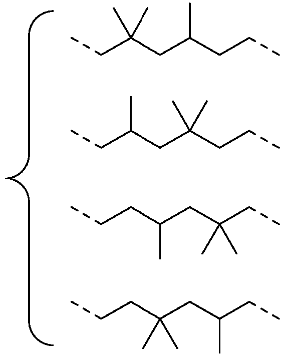
R² is a C₂-C₁₁, preferably a C₂-C₈ and most preferably a C₂-C₆ alkylidene group, that can be branched or linear and that can be interrupted by one or more, preferably a single, O atoms,
R³ O, NH or NR⁶, in which R⁶ is a linear and branched C₁-C₆ alkyl group,
R⁴ is one of the following groups: 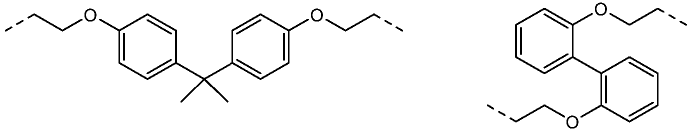 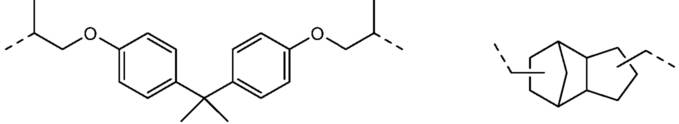 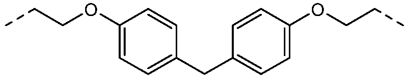
R⁵ is H or CH₃, and
m is a value from 0.5 to 3.0.

2. The urethane di(meth)acrylate macromonomer according to claim 1, wherein the variables of Formula I have the following meanings:
R¹ is 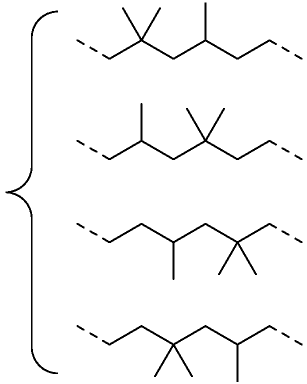
R² is a linear C₂-C₆, preferably a linear C₂-C₄ alkylidene group,
R³ is O,
R⁴ is one of the following groups: 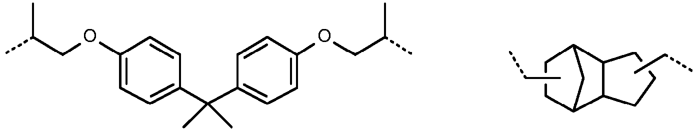
R⁵ is CH₃,
m is a value from 0.5 to 3.0.

3. The urethane di(meth)acrylate macromonomer according to claim 1 or 2, wherein *m* is a value of from 0.6 to 2.0, preferably 0.7 to 1.6 and more preferably 0.9 to 1.6.

4. The urethane di(meth)acrylate macromonomer according to any one of claims 1 to 3 having a number-average molar mass of from 800 to 2000 g/mol, more preferably of from 800 to 1800 g/mol and most preferably 800 to 1600 g/mol.

5. A radically polymerizable dental material, comprising
(a) 15 to 75 wt.% of at least one aromatic, bicyclic or tricyclic mono(meth)acrylate,
(b) 15 to 75 wt.% of at least one urethane di(meth)acrylate macromonomer according to any one of claims 1 to 4,
(c) 0 to 30 wt.% of one or more di(meth)acrylate monomers,
(d) 0.1 to 10 wt.%, preferably 2 to 10 wt.%, of at least one (PCL)_{q}-b-(PDMS)ᵣ-b-(PCL)_{q} block copolymer having a number-average molar mass of 2000 to 10000 g/mol, wherein (PCL)_{q} represents polycaprolactone, which is made up of q caprolactone monomer units, and (PDMS)ᵣ represents poly(dimethylsiloxane), which is made up of r dimethylsiloxane monomer units and the letter b stands for *block,*
(e) 0.1 to 3.0 wt.% of at least one initiator for the radical polymerization.

6. The dental material according to claim 5, which comprises as **component (a)** at least one aromatic, bicyclic or tricyclic monomethacrylate of Formula II: 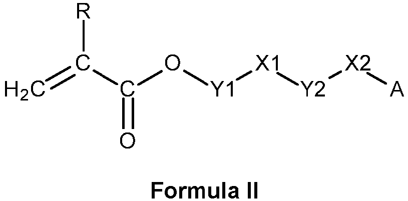 in which the variables have the following meanings:
A an aromatic group with 6 to 15 carbon atoms or a bicyclic or tricyclic aliphatic group with 7 to 10 carbon atoms, wherein A can be unsubstituted or substituted by one or more C₁-C₅ alkyl groups, C₁-C₅ alkoxy groups and/or chlorine atoms;
R methyl or preferably H;
X¹, X² independently of each other is in each case are absent or an ether, ester or urethane group, wherein X¹ is absent if Y¹ is absent and wherein X² is absent if Y² is absent;
Y¹, Y² independently of each other is in each case absent or a branched or preferably linear aliphatic hydrocarbon radical with 1 to 10 carbon atoms, which can be interrupted by 1 to 3 oxygen atoms.

7. The dental material according to claim 6, which comprises as component (a) 2-phenoxyethyl (meth)acrylate, 2-(o-biphenyloxy)ethyl (meth)acrylate, 2-hydroxy-3-phenoxypropyl (meth)acrylate, phenethyl (meth)acrylate, 2-[(benzyloxycarbonyl)-amino]-ethyl (meth)acrylate, 2-[(benzylcarbamoyl)-oxy]-ethyl (meth)acrylate, 1-phenoxypropan-2-yl (meth)acrylate and 2-(p-cumylphenoxy)-ethyl (meth)acrylate, 2-(benzyloxy)ethyl (meth)acrylate, 3-phenoxybenzyl (meth)acrylate, phenoxypropyl (meth)acrylate, 2-benzyloxyethyl (meth)-acrylate, 2-benzoyloxyethyl (meth)acrylate, 2-(meth)acryloyloxybenzoic acid methyl ester,
preferably octahydro-4,7-methano-1H-indenyl)methyl (meth)acrylate, (octahy-dro-4,7-methano-1H-indenyl) (meth)acrylate, isobornyl (meth)acrylate or a mixture thereof,
more preferably (octahydro-4,7-methano-1H-indenyl)methyl acrylate (OMIMA), isobornyl acrylate (IBOA) or a mixture thereof.

8. The dental material according to claim 5, which comprises
(a) 20 to 70 wt.% and preferably 25 to 65 wt.% of (octahydro-4,7-methano-1H-indenyl)methyl acrylate (OMIMA), isobornyl acrylate (IBOA) or a mixture thereof,
(b) 20 to 70 wt.% and preferably 25 to 65 wt.% of at least one urethane di(meth)acrylate macromonomer according to any one of claims 1 to 4,
(c) 0 to 20 wt.% and preferably 0 to 10 wt.% of one ore di(meth)acrylate monomers,
(d) 3 to 9 wt.% and preferably 4 to 8 wt.% of at least one (PCL)_{q}-b-(PDMS)ᵣ-b-(PCL)_{q} block copolymer having a number-average molar mass of 2000 to 10000 g/mol, and
(e) 0.3 to 2.5 wt.% and preferably 0.5 to 2.0 wt.% of at least one initiator for the radical polymerization,
in each case relative to the total mass of the material.

9. The dental material according to one of claims 5 to 8, which comprises as **component (b)** at least one urethane dimethacrylate macromonomer, which is obtainable by reacting a diisocyanate with a diol and then reacting the α,ω-isocyanate-functionalized urethane macromonomer with a compound bearing one hydroxy group and one radically polymerizable group, preferably HEMA or HPMA.

10. The dental material according to one of claims 5 to 9, which comprises as **component (d)** at least one block copolymer according to the formula (PCL)_{q}-b-(PDMS)ᵣ-b-(PCL)_{q} having a number-average molar mass of from 2000 to 10000 g/mol, wherein the variables q and r preferably have the following meanings:
q in each case is a number from 4 to 30, preferably 7 to 20, and
r is a number from 15 to 60, preferably 20 to 45 and
r/q is within a range of 1.75 to 4.

11. The dental material according to one of claims 5 to 10, which comprises as **component (e)** at least one photoinitiator, which is preferably selected from an α-diketone or a derivative thereof, such as 9,10-phenanthrenequinone, 1-phenyl-propane-1,2-dione, diacetyl or 4,4'-dichlorobenzil, camphorquinone (CQ), 2,2-dimethoxy-2-phenylacetophenone or an α-diketone in combination with an amine as reducing agent, such as e.g. 4-(dimethylamino)-benzoic acid ester (EDMAB), N,N-dimethylaminoethyl methacrylate, N,N-dimethyl-sym.-xylidine or triethanolamine, monoacyltrialkyl-, diacyldialkyl-, tetraacylgermanium, a tetraacylstannane, benzoyltrimethylgermanium, dibenzoyldiethylgerma-nium, bis(4-methoxybenzoyl)diethylgermanium, tetrakis(2-methylbenzoyl)-germane or tetrakis(mesitoyl)stannane or a mixture thereof,
a Norrish type I photoinitiator, which is preferably selected from acetophenones, e.g. 2,2-diethoxy-1-phenylethanone, benzoin ethers e.g. Irgacure 651 (benzil dimethyl ketal), hydroxyalkylphenylacetophenones, e.g. Irgacure 184 (1-hydroxycyclohexyl phenyl ketone), an acyl- or bisacylphosphine oxide, e.g. Irgacure TPO (2,4,6-trimethylbenzoyldiphenylphosphine oxide), Irgacure TPO-L (ethyl phenyl(2,4,6-trimethylbenzoyl)phosphinate), TMO (2,4,6-trimethylbenzoyl)bis(p-tolyl)phosphine oxide or Irgacure 819 (bis(2,4,6-trimethylbenzoyl)phenylphosphine oxide), 2-benzyl-2-(dimethylamino)-4'-morpholino-butyrophenone (Irgacure 369) and/or 1-butanone-2-(dimethylamino)-2-(4-methylphenyl)methyl-1-4-(4-morpholinyl)phenyl (Irgacure 379).

12. The dental material according to one of claims 5 to 11, with the following composition:
(a) 15 to 75 wt.%, preferably 20 to 70 wt.%, particularly preferably 25 to 65 wt.% of at least one mono(meth)acrylate (a),
(b) 15 to 75 wt.%, preferably 20 to 70 wt.% and particularly preferably 25 to 65 wt.% of at least one urethane di(meth)acrylate macromonomer according to any one of claims 1 to 4, wherein *m* is a value of from 0.6 to 2.0,
(c) 0 to 30 wt.%, preferably 0 to 20 wt.% and particularly preferably 0 to 10 wt.% of one or more di(meth)acrylate monomers,
(d) 0.1 to 10 wt.% or 2 to 10 wt.%, preferably 3 to 9 wt.%, particularly preferably 4 to 8 wt.% of at least one (PCL)_{q}-b-(PDMS)ᵣ-b-(PCL)_{q} block copolymer having a number-average molar mass of 2000 to 10000 g/mol,
(e) 0.1 to 3.0 wt.%, preferably 0.3 to 2.5 wt.% and particularly preferably 0.5 to 2.0 wt.% of at least one initiator for the radical polymerization,
(f) 0 to 15 wt.%, preferably 0 to 5 wt.% and particularly preferably 0 wt.% of core-shell polymer particles,
(g) 0 to 20 wt.%, preferably 0 to 15 wt.% and particularly preferably 0 to 10 wt.% of filler,
(h) 0 to 1.0 wt.%, preferably 0 to 0.7 wt.% and particularly preferably 0 to 0.5 wt. % of UV absorber,
(i) 0 to 0.5 wt.%, preferably 0 to 0.1 wt.% and particularly preferably 0 to 0.05 wt.% of optical brightener and
(j) 0 to 15 wt.%, preferably 0 to 10 wt.% and particularly preferably 0.05 to 5 wt.% of further additives.
in each case relative to the total mass of the material.

13. A process for the production of dental shaped parts in which
(i) a virtual image of the tooth situation is created by direct or indirect digitization of the tooth to be restored or of the teeth to be restored on a computer,
(ii) then a model of the dental restoration or prosthesis is constructed on the computer on the basis of this image,
(iii) a dental material in accordance with one of claims 5 to 12 is then polymerized in layers by selective light irradiation in order to form the dental restoration and
(iv) the workpiece is then cleaned, optionally by treatment with a solvent, and
(v) the workpiece is then optionally further cured by irradiating with light and/or by heating to a temperature above 50°C.

14. The use of a urethane di(meth)acrylate macromonomer according to any one of claims 1 to 4 for increasing the fracture toughness and/or fracture work of radically polymerizable materials.

15. The use of a material in accordance with any one of claims 5 to 12 as dental material or for the production or repair of dental shaped parts.
